# EUROPEAN PATENT APPLICATION

(11) **EP 4 502 162 A1**
(43) Date of publication of application: **05.02.2025**
(21) Application number: 23780559.3
(22) Date of filing: 28.03.2023
(51) Int. Cl.: C12N 15/62, A61K 35/12, A61P 35/00, C07K 14/715, C07K 19/00, C12N 5/0783, C12N 5/10, C12N 15/12, C12N 15/63

(54) **SELECTIVE REGULATORY GENE (SRG) SYSTEM FOR GENETICALLY MODIFIED IMMUNE CELL THERAPY**

(30) Priority: 29.03.2022 JP 2022052759
(71) Applicant: Jichi Medical University, Tokyo 102-0093 (JP); Takara Bio Inc., Kusatsu-shi Shiga 525-0058 (JP)
(72) Inventor: UCHIBORI, Ryosuke, Shimotsuke-shi, Tochigi 329-0498 (JP); OHMINE, Ken, Shimotsuke-shi, Tochigi 329-0498 (JP); OZAWA, Keiya, Shimotsuke-shi, Tochigi 329-0498 (JP)
(74) Representative: Schnappauf, Georg
(86) International application number: PCT/JP2023/012578
(87) International publication number: WO 2023/190550

(57) **Abstract**

The present invention provides a chimeric receptor comprising: a first polypeptide which has (i) a first extracellular region binding to a ligand, (ii) a first transmembrane region, and (iii) a first intracellular region derived from an IL-2 receptor β chain, and in which a mutation is introduced to a tyrosine residue in the first intracellular region; and a second polypeptide which has (iv) a second extracellular region binding to a ligand, (v) a second transmembrane region, and (vi) a second intracellular region derived from an IL-2 receptor y chain.

## Description

### TECHNICAL FIELD

The present invention relates to a chimeric receptor that is useful for improving the *in vitro* proliferation ability, *in vivo* proliferation ability, and *in vivo* survival ability of immune cells such as T cells.

### BACKGROUND ART

As a therapeutic strategy for tumors, it is expected that T cells targeting tumor cells can be produced by introducing into T cells a nucleic acid encoding a chimeric antigen receptor (CAR) that binds to a specific antigen present on the surface of tumor cells, or a nucleic acid encoding a T cell receptor (TCR) that recognizes tumor cells. Herein, T cells into which a nucleic acid encoding CAR has been introduced are called CAR-T cells, and T cells into which a nucleic acid encoding TCR has been introduced are called TCR-T cells.

Currently, CAR-T cell or TCR-T cell therapies targeting various tumor antigens, such as CD19, BCMA, WT1, MART1, gp100, CEA, and mHAG HA-2 antigens, have been developed. For example, therapy using CD19-specific CAR-T cells has demonstrated remarkable therapeutic effects against B-cell tumors. However, long-term follow-up has revealed tumor recurrence, and treatment outcomes are far from satisfactory, calling for further improvements. One of the reasons for tumor recurrence after treatment with CAR-T cells is the inability of CAR-T cells to survive in the body for a long period.

Interleukin 2 (hereinafter, referred to as IL-2) may be used to maintain the proliferation and antitumor activity of exogenously administered T cells in the body. However, administration of IL-2 induces side effects. In addition, IL-2 reacts with all T cells expressing IL-2 receptors to activate not only the administered T cells, but also endogenous regulatory T cells, thereby weakening the immune response.

As a known means for overcoming the IL-2 requirement of T cells, erythropoietin, which is a low-toxicity cytokine, is used as a ligand instead of IL-2. In 1995, Minamoto et al. developed a chimeric receptor comprising the extracellular domain of an erythropoietin receptor and the intracellular domain of an IL-2 receptor subunit β-chain or γ-chain (Non-Patent Document 1).

In 2020, Campana et al. reported the EpoRm-CAR system, which co-expresses an erythropoietin receptor mutant and CAR (Non-Patent Document 2). This system was developed to enhance the *in vivo* survival of T cells co-expressing an erythropoietin receptor mutant and CAR (hereinafter, EpoRm-CAR-T cells) through erythropoietin stimulation. However, *in vitro* erythropoietin stimulation caused EpoRm-CAR-T cells to proliferate only about twice as much as control cells. In addition, *in vivo* treatment experiments showed that proliferation of tumor cells was suppressed only when mice injected with EpoRm-CAR-T cells in advance were inoculated with the cancer cells. Thus, the effectiveness of the EpoRm-CAR system is thought to be insufficient.

### PRIOR ART DOCUMENT

### NON-PATENT DOCUMENT

Non-patent Document 1: Blood, 1995 Sep 15; 86(6): 2281-7
Non-patent Document 2: Blood, 2020 Feb 27; 135(9): 668-679

### SUMMARY OF THE INVENTION

### PROBLEMS TO BE SOLVED BY THE INVENTION

As described above, with the conventional CAR-T therapy, long-term follow-up has revealed tumor recurrence, and treatment outcomes are far from satisfactory. Thus, further improvements are needed. Specifically, there is a need for a novel method for allowing administered T cells to survive in the body of a patient for an extended period of time.

### SOLUTIONS TO THE PROBLEMS

As a result of intensive research to solve the above problems, the present inventors found a method for selectively controlling the proliferation and survival of T cells ex vivo and *in vivo,* and completed the present invention.

That is, the present invention relates to:
[1] A chimeric receptor comprising a first polypeptide and a second polypeptide, the first polypeptide comprising:
   (i) a first extracellular region that binds to a ligand,
   (ii) a first transmembrane region, and
   (iii) a first intracellular region derived from an IL-2 receptor β chain, wherein a mutation is introduced at one or more tyrosine residues in the first intracellular region; and the second polypeptide comprising:
   (iv) a second extracellular region that binds to a ligand,
   (v) a second transmembrane region, and
   (vi) a second intracellular region derived from an IL-2 receptor γ chain;
[2] The chimeric receptor according to [1], wherein the one or more tyrosine residues in the first intracellular region derived from an IL-2 receptor β chain described in (iii) are selected from the group consisting of tyrosine residues corresponding to positions 381, 384 and 387 in the amino acid sequence of an IL-2 receptor β chain (RefSeq NP_000869.1);
[3] The chimeric receptor according to [2], wherein the one or more tyrosine residues in the first intracellular region derived from an IL-2 receptor β chain described in (iii) are tyrosine residues corresponding to positions 381, 384 and 387 in the amino acid sequence of an IL-2 receptor β chain (RefSeq NP_000869.1);
[4] The chimeric receptor according to any one of [1] to [3], wherein the mutation is a substitution of the tyrosine residue with a phenylalanine residue;
[5] The chimeric receptor according to any one of [1] to [4], wherein the first extracellular region that binds to a ligand of (i) and/or the second extracellular region that binds to a ligand of (iv) is an extracellular region derived from an erythropoietin receptor;
[6] The chimeric receptor according to any one of [1] to [5], wherein the first transmembrane region of (ii) is a transmembrane region derived from an IL-2 receptor β chain, and the second transmembrane region of (v) is a transmembrane region derived from an IL-2 receptor γ chain;
[7] The chimeric receptor according to any one of [1] to [6], wherein the second polypeptide further comprises an intracellular region derived from CD40;
[8] A nucleic acid encoding the chimeric receptor according to any one of [1] to [7];
[9] A vector comprising the nucleic acid of [8];
[10] The vector according to [9], which is selected from the group consisting of a plasmid vector, a viral vector, and an artificial chromosome;
[11] The vector according to [9] or [10], further comprising a nucleic acid encoding a chimeric antigen receptor or a foreign T cell receptor that recognizes a target cell;
[12] A cell expressing the chimeric receptor according to any one of [1] to [7];
[13] The cell according to [12], which further expresses a chimeric antigen receptor or a foreign T cell receptor that recognizes a target cell;
[14] The cell according to [12] or [13], which is an immune cell;
[15] The cell according to [14], which is selected from the group consisting of a T cell, a tumor-infiltrating lymphocyte, an NK cell, and an NK-T cell;
[16] The cell according to any one of [12] to [15], which is a human-derived cell;
[17] A method for producing a cell expressing a chimeric receptor, the method comprising a step of introducing the nucleic acid according to [8] into a cell;
[18] The method according to [17], which comprises a step of introducing the vector according to any one of [9] to [11] into a cell;
[19] The method according to [17] or [18], which further comprises a step of isolating and/or expanding the cell expressing a chimeric receptor;
[20] The method according to any one of [17] to [19], which comprises a step of culturing the cell into which the nucleic acid encoding the chimeric receptor has been introduced in the presence of the ligand;
[21] The method according to [20], which comprises a step of culturing the cell into which the nucleic acid encoding the chimeric receptor comprising the extracellular domain of the erythropoietin receptor has been introduced in the presence of erythropoietin;
[22] A pharmaceutical composition comprising the cell according to any one of [12] to [16] as an active ingredient;
[23] The pharmaceutical composition according to [22], wherein the cell further expresses a chimeric antigen receptor or a foreign T cell receptor that recognizes a target cell;
[24] The pharmaceutical composition according to [23], wherein the target cell is a tumor cell, and the pharmaceutical composition comprises, as an active ingredient, the cell expressing a chimeric antigen receptor or a foreign T cell receptor that recognizes a tumor antigen;
[25] A method for preventing or treating a disease, the method comprising administering the pharmaceutical composition according to any one of [22] to [24] to a subject; and
[26] A method for reducing target cells in a subject, the method comprising administering the pharmaceutical composition according to any one of [22] to [24] to a subject in need thereof.

### EFFECTS OF THE INVENTION

The present invention provides a chimeric receptor that is useful in the field of gene-modified immune cell therapy targeting antigens such as tumor antigens, a nucleic acid encoding the chimeric receptor, a vector containing the nucleic acid, a cell expressing the chimeric receptor, a method for producing the cell, a pharmaceutical composition containing the cell as an active ingredient, and a method for preventing (including prevention of recurrence, the same applies below) or treating a disease, which is characterized by administering the pharmaceutical composition to a subject. CAR-T cells or TCR-T cells into which the chimeric receptor of the present invention has been introduced can survive in the body of a patient for a long period of time and exhibit high therapeutic efficacy.

### BRIEF DESCRIPTION OF THE DRAWINGS

[FIG. 1] FIG. 1 shows a method for constructing plasmid pEX-A2J2/TEGPEB.
[FIG. 2] FIG. 2 shows a method for constructing retroviral plasmid pMEI-5/ZG-SRG_YYY.
[FIG. 3] FIG. 3 shows a method for constructing retroviral plasmid pMEI-5/ZG-SRG_FYY.
[FIG. 4] FIG. 4 shows a method for constructing retroviral plasmid pMEI-5/ZG-SRG_YFY.
[FIG. 5] FIG. 5 shows a method for constructing retroviral plasmid pMEI-5/ZG-SRG_YYF.
[FIG. 6] FIG. 6 shows a method for constructing retroviral plasmid pMEI-5/ZG-SRG FFY.
[FIG. 7] FIG. 7 shows a method for constructing retroviral plasmid pMEI-5/ZG-SRG_YFF.
[FIG. 8] FIG. 8 shows a method for constructing retroviral plasmid pMEI-5/ZG-SRG_FYF.
[FIG. 9] FIG. 9 shows a method for constructing retroviral plasmid pMEI-5/ZG-SRG_FFF.
[FIG. 10] FIG. 10 shows a method for constructing retroviral plasmid pMEI-5/SRG_YYY.
[FIG. 11] FIG. 11 shows a method for constructing retroviral plasmid pMEI-5/SRG_FFF.
[FIG. 12] FIG. 12 shows a method for constructing retroviral plasmid pMEI-5/EL-SRG_FFF.
[FIG.13] FIG. 13 shows a method for constructing retroviral plasmid pMEI-5/FMC28z.
[FIG. 14] FIG. 14 shows a method for constructing retroviral plasmid pMEI-5/FMC28z-SRG_FFF.
[FIG. 15] FIG. 15 shows a method for constructing retroviral plasmid pMEI-5/ZG-EpoRm.
[FIG. 16] FIG, 16 shows a flowchart of the production and expansion of transduced cells in Example 3-(2).
[FIG. 17-1] FIG. 17-1 shows the growth curve of transduced cells in Example 3-(2).
[FIG. 17-2] FIG. 17-2 shows the growth curve of transduced cells in Example 3-(2).
[FIG. 18] FIG. 18 shows ZsGreen1 positive rates in transduced cells in Example 3-(2).
[FIG. 19] FIG. 19 shows growth curves of transduced cells in Example 4.
[FIG. 20} FIG. 20 shows the growth curve of transduced cells in Example 5.
[FIG. 21] FIG. 21 shows expansion conditions in Example 6.
[FIG. 22-1] FIG. 22-1 shows the growth curves of transduced cells in Example 6.
[FIG. 22-2] FIG. 22-2 shows the growth curves of transduced cells in Example 6.
[FIG. 23-1] FIG. 23-1 shows ZsGreen1 positivity rates in transduced cells in Example 6.
[FIG. 23-2] FIG. 23-2 shows ZsGreen1 positivity rates in transduced cells in Example 6.
[FIG. 24] FIG. 24 shows a flowchart for the production of transduced cells and a flowchart for the measurement of *in vivo* bioluminescence imaging, in Example 7-(1).
[FIG. 25] FIG. 25 shows *in vivo* bioluminescence imaging in Example 7-(1).
[FIG. 26-1] FIG. 26-1 shows graphs of the quantified luminescence intensity in Example 7-(1).
[FIG. 26-2] FIG. 26-2 shows graphs of the quantified luminescence intensity in Example 7-(1).
[FIG. 27] FIG. 27 shows hematocrit values of whole blood in Example 7-(1).
[FIG. 28] FIG. 28 shows a flowchart for the production of transduced cells and a flowchart for the measurement of in *vivo* bioluminescence imaging, in Example 7-(2).
[FIG. 29] FIG. 29 shows *in vivo* bioluminescence imaging in Example 7-(2).
[FIG. 30] FIG. 30 shows graphs of the quantified luminescence intensity in Example 7-(2).
[FIG. 31] FIG. 31 shows hematocrit values of whole blood in Example 7-(2).
[FIG. 32] FIG. 32 shows CAR and SRG (EpoR) positivity rates in Example 8.
[FIG. 33] FIG. 33 shows ratios of CD4 to CD8 in Example 8.
[FIG. 34-1] FIG 34-1 shows ratios of CD4 and CD8 T cell subsets in Example 8.
[FIG. 34-2] FIG 34-2 shows ratios of CD4 and CD8 T cell subsets in Example 8.
[FIG. 35] FIG. 35 shows measurement of cytotoxic activity in Example 9-(1).
[FIG. 36-1] FIG. 36-1 shows results of measurement of IL-2 and IFN-γ production in Example 9-(1).
[FIG. 36-2] FIG. 36-2 shows results of measurement of IL-2 and IFN-γ production in Example 9-(1).
[FIG. 37] FIG. 37 show a flowchart for the production of transduced cells and a flowchart for the measurement of in *vivo* bioluminescence imaging, in Example 10.
[FIG. 38] FIG. 38 shows *in vivo* bioluminescence imaging in Example 10.
[FIG. 39] FIG. 39 shows graphs of quantified luminescence intensity in Example 10.
[FIG. 40] FIG. 40 shows Kaplan-Meier curves in Example 10.
[FIG. 41] FIG. 41 shows a method for constructing retroviral plasmid pMEI-5/ZG-SRG_FFF40.
[FIG. 42] FIG. 42 shows a method for constructing retroviral plasmid pMEI-5/SRG_FFF40.
[FIG. 43] FIG. 43 shows a method for constructing retroviral plasmid pMEI-5/EL-SRG_FFF40.
[FIG. 44] FIG. 44 shows a method for constructing retroviral plasmid pMEI-5/FMC28z-SRG_FFF40.
[FIG. 45] FIG. 45 shows measurement of cytotoxic activity in Example 12.
[FIG. 46] FIG. 46 shows Kaplan-Meier curves in Example 13.

### MODE FOR CARRYING OUT THE INVENTION

The present invention will be described in detail below.

### (1) Chimeric receptor of the present invention

The chimeric receptor of the present invention comprises a first polypeptide and a second polypeptide, the first polypeptide comprising (i) a first extracellular region that binds to a ligand, (ii) a first transmembrane region, and (iii) a first intracellular region derived from an IL-2 receptor β chain, wherein a mutation(s) is introduced in the first intracellular region; and the second polypeptide comprising (iv) a second extracellular region that binds to a ligand, (v) a second transmembrane region, and (vi) a second intracellular region derived from an IL-2 receptor γ chain.

The first polypeptide constituting the chimeric receptor of the present invention comprises, in a direction from the N-terminus to the C-terminus, (i) a first extracellular region that binds to a ligand, (ii) a first transmembrane region, and (iii) a first intracellular region derived from an IL-2 receptor β chain. The second polypeptide comprises, in a direction from the N-terminus to the C-terminus, (iv) a second extracellular region that binds to a ligand, (v) a second transmembrane region, and (vi) a second intracellular region derived from an IL-2 receptor γ chain.

Furthermore, both the polypeptides may have a signal peptide at their N-termini. The signal peptide may be, for example, a signal peptide originally possessed by an extracellular region derived from any polypeptide which is contained in the chimeric receptor of the present invention as the first or second extracellular region, or may be a signal peptide derived from other secretory proteins or membrane proteins.

The first extracellular domain and the second extracellular domain may be different from each other or may be the same. Moreover, these two extracellular regions may be different regions derived from the same polypeptide. These two extracellular regions may be appropriately selected from extracellular regions described below. Furthermore, the first transmembrane region and the second transmembrane region may be different from each other or may be the same. Furthermore, these two transmembrane regions may be different regions derived from the same polypeptide. These two transmembrane regions may be appropriately selected from transmembrane regions described below.

As used herein, the ligand is a molecule that can be recognized by or bind to a specific polypeptide, i.e., the "extracellular region" as described below, and is not particularly limited. Examples of the ligand include nucleic acids, sugar chains, lipids, peptides, and proteins. For example, when the extracellular region is derived from a receptor, a ligand for the receptor can be used as the ligand in the present invention, and when the extracellular region is an antigen-binding domain of an antibody, an antigen can be used as the ligand in the present invention. Examples of the nucleic acid include a short-chain nucleic acid, a long-chain nucleic acid, a single-stranded nucleic acid, a double-stranded nucleic acid, DNA, and RNA. Examples of DNA include double-stranded DNA, single-stranded DNA, and cDNA. Examples of RNA include mRNA. The short-chain nucleic acid is defined as a nucleic acid of 2 to 100 bases, and the long-chain nucleic acid is defined as a nucleic acid of more than 100 bases.

Examples of protein ligands include cytokines, for example interleukins (IL-1, IL-3, IL-4, IL-6, IL-7, IL-15, IL-21), hematopoietic factors (erythropoietin, thrombopoietin, G-CSF, GM-CSF, etc.), stem cell factor (SCF), and cell growth factors (FGF, EGF, IGF, HGF, PDGF, NGF, TGF), and T cell activation regulators (PD-L1, PD-L2, CD80, CD86, Ceacam-1, Galectin-3, Galectin-9, FGL-1, CD112, CD155, HVEM, CD40, OX40L, 4-1BBL, GITRL, CD70, etc.).

Other examples of the ligand include agonists and antagonists for cell membrane receptors, toxins and venoms, viral epitopes, hormones (e.g., opioid hormones, steroid hormones, etc.), peptides, enzymes, enzyme substrates, coenzymes, drugs, lectins, sugars, oligosaccharides, antigens, monoclonal antibodies, cells, bacteria, viruses, avidin, etc.

As the ligand in the present invention, a ligand that does not interact or shows no significant interaction with cells on which the chimeric receptor of the present invention is to be expressed and with cells that may coexist with the cells on which the chimeric receptor of the present invention is to be expressed is selected. For example, when the chimeric receptor is to be expressed on T cells, the ligand is preferably a cytokine for which a receptor is not highly expressed on T cells, and more preferably erythropoietin.

"Extracellular region that binds to a ligand"

As used herein, the term "(i) a first extracellular region that binds to a ligand" or " (iv) a second extracellular region that binds to a ligand" (hereinafter collectively referred to as the "extracellular region that binds to a ligand") refer to a region containing a proteinaceous molecule or a part thereof that can bind to a ligand. Examples of the extracellular region that binds to a ligand include an extracellular region derived from a receptor, and an antigen-binding domain of an antibody. The extracellular region binds to and interacts with a ligand, thereby ligand specificity is conferred to a cell expressing the chimeric receptor of the present invention.

The extracellular region is also called the "extracellular domain". As used herein, the term "domain" refers to a region within a polypeptide that is folded into a specific structure independently of other regions.

As used herein, the "extracellular region that binds to a ligand" may be selected from those capable of binding to the ligand to be used. Examples of the extracellular region that binds to a ligand include an extracellular region derived from a cytokine receptor, an extracellular region derived from a growth factor receptor, an extracellular region derived from a receptor tyrosine kinase, and an extracellular region derived from a receptor for a T-cell activation regulator, as well as their mutants having the same function as these sequences. In the present invention, an extracellular region derived from a cytokine receptor is preferably used. Examples of the cytokine receptor include, but not limited to, an interleukin 1 receptor (IL-1RI), IL-1 receptor accessory protein (IL-1RAcP), IL-3Rα, IL-3Rβ (common β chain), IL-4Rα, IL-5Rα, IL-6Rα, gp130, IL-7Rα, IL-15Rα, IL-21R, an erythropoietin receptor (EpoR), a thrombopoietin receptor (TpoR), a granulocyte colony-stimulating factor receptor (G-CSFR), a granulocyte-macrophage colony-stimulating factor receptor (GM-CSFR), and a growth hormone receptor (GHR). Examples of the growth factor receptor include, but not limited to, FGF receptor 1, FGF receptor 2, FGF receptor 3, an EGF receptor, an IGF-1 receptor, a MET receptor, a PDGF receptor, an NGF receptor, a TGF-β type I receptor (TβRI), and a TGF-β type II receptor (TβRII). Examples of the receptor tyrosine kinase include, but not limited to, EPH receptor A4 (EPHA4), and c-kit. Examples of the receptor for a T-cell activation regulator include, but not limited to, PD-1, CTLA-4, TIM-3, LAG-3, CD27, CD28, CD226, CD40L, GITR, OX-40, TIGIT, ICOS, 4-1BB, and BTLA.

Another example of the "extracellular region that binds to a ligand" used in the present invention is an antigen-binding domain of an antibody. The "antigen-binding domain" refers to an antigen-binding portion of an antibody capable of binding to an antigen. Examples of the antigen-binding domain include a Fab' fragment, a Fab fragment, an Fv fragment, and a single chain variable fragment (scFv).

Furthermore, the "extracellular region that binds to a ligand" of the present invention may be derived from a TCR (TCRα, TCRβ, TCRγ, TCRδ), a CD4 ectodomain, CD8α, CD8β, CD11A, CD11B, CD11C, CD18, CD29, CD49A, CD49B, CD49D, CD49E, CD49F, CD61, CD41, and/or CD51. For example, the CD4 ectodomain can recognize HIV-infected cells.

As used herein, the "extracellular region that binds to a ligand" is preferably an extracellular region derived from a receptor, more preferably an extracellular region derived from a cytokine receptor, and most preferably an extracellular region derived from an erythropoietin receptor.

The "(i) first extracellular region that binds to a ligand" and the "(iv) second extracellular region that binds to a ligand" are independently selected and may be the same extracellular region or different types of extracellular regions. For example, one of the "(i) first extracellular region that binds to a ligand" and the "(iv) second extracellular region that binds to a ligand" may be an extracellular region derived from an erythropoietin receptor, and the other may be another type of extracellular region, or both of the "(i) first extracellular region that binds to a ligand" and the "(iv) second extracellular region that binds to a ligand" may be extracellular regions derived from an erythropoietin receptor.

### "Transmembrane region"

The transmembrane region is also called the "transmembrane domain", and generally refers to a region of a membrane protein that is embedded in the cell membrane. As used herein, the "(ii) first transmembrane region" and "(v) second transmembrane region" (hereinafter collectively referred to as the "transmembrane region") may be derived from a naturally occurring polypeptide or may be artificially designed. The transmembrane region derived from a naturally occurring polypeptide can be obtained from a membrane-bound or transmembrane protein. Examples of the transmembrane region derived from a naturally occurring polypeptide include transmembrane regions of an interleukin 1 receptor (IL-1RI), an IL-1 receptor accessory protein (IL-1RAcP), an IL-2 receptor α chain, an IL-2 receptor β chain, an IL-2 receptor γ chain, IL-3Rα, IL-3Rβ (common β chain), IL-4Rα, IL-5Rα, IL-6Rα, gp130, IL-7Rα, IL-15Rα, IL-21R, an erythropoietin receptor (EpoR), a thrombopoietin receptor, a granulocyte colony-stimulating factor receptor (G-CSFR), a granulocyte-macrophage colony-stimulating factor receptor (GM-CSFR), a growth hormone receptor (GHR), a fibroblast growth factor receptor (FGFR1, FGFR2, FGFR3), an epithelial growth factor receptor (EGFR), an insulin-like growth factor 1 receptor (IGF-1R), a MET receptor, a platelet-derived growth factor receptor (PDGFR), a nerve growth factor receptor (NGFR), a TGF-β type I receptor (TβRI), a TGF-β type II receptor (TβRII), erythropoietin-producing hepatocyte receptor A4 (EPHA4), c-kit, T cell receptor α, a T cell receptor β chain, a CD3 zeta chain, CD28, CD3ε, CD45, CD4, CD5, CD8, CD9, CD16, CD22, CD33, CD37, CD64, CD80, CD86, CD134, CD137, ICOS, CD154, and GITR. The artificially designed transmembrane region is a polypeptide mainly comprising hydrophobic residues such as leucine and valine. For example, a triplet of phenylalanine, tryptophan and valine may be found at each end of the synthetic transmembrane region. Optionally, a short oligopeptide linker or a polypeptide linker, for example a linker of 2-10 amino acids in length, can be placed between the first transmembrane region and the first intracellular region described herein. Preferably, a linker sequence comprising a glycine-serine contiguous sequence is used.

As used herein, the "(ii) first transmembrane region" contained in the first polypeptide is preferably a transmembrane region derived from an IL-2 receptor β chain. The "(v) second transmembrane region" contained in the second polypeptide is preferably a transmembrane region derived from an IL-2 receptor γ chain.

### "(iii) First intracellular region derived from an IL-2 receptor beta chain"

The intracellular region contained in the first polypeptide constituting the chimeric receptor of the present invention is capable of transmitting a signal into the cell when the extracellular region present in the same molecule binds to (interacts with) a ligand.

As used herein, the "IL-2 receptor" means a receptor for IL-2. The native human IL-2 receptor is composed of three cell membrane surface proteins: an α chain (also known as CD25, 55 kDa, 251 amino acid residues), a β chain (also known as CD122, 75 kDa, 525 amino acid residues), and a γ chain (also known as CD132, 64 kDa, 369 amino acid residues). In nature, a complex of these proteins forms a non-covalent bond with an IL-2 molecule to transmit a signal into the cell. The β and γ chains belong to type I interleukin receptor family. The receptor proteins form a dimer to transmit a signal into the cell, and normally they move freely on the cell membrane as monomers (the so-called fluid mosaic model). However, when a ligand approaches, the receptor proteins dimerize to become capable of functioning as the receptor.

The origin of the IL-2 receptor used in the present invention is not particularly limited, and an IL-2 receptor derived from a mammal, for example, a human IL-2 receptor, or an IL-2 receptor derived from a non-human mammal such as a monkey, mouse, rat, pig, horse, or dog can be used. The IL-2 receptor used in the present invention is preferably a human IL-2 receptor.

The "(iii) first intracellular region derived from an IL-2 receptor β chain" used in the present invention may be the entire intracellular region of a native IL-2 receptor β chain, or a partial intracellular region of a native IL-2 receptor β chain. An example of the entire intracellular region of a native IL-2 receptor β chain is the amino acid sequence from amino acids 266 to 551 in the wild-type human IL-2 receptor β chain amino acid sequence (RefSeq NP_000869.1). Examples of the partial intracellular region of a native IL-2 receptor β chain include amino acid sequences from amino acids 266 to 541, from amino acids 266 to 531, from amino acids 266 to 521, from amino acids 266 to 511, from amino acids 266 to 501, from amino acids 266 to 491, from amino acids 266 to 481, from amino acids 266 to 471, from amino acids 266 to 461, from amino acids 266 to 451, from amino acids 266 to 441, from amino acids 266 to 431, from amino acids 266 to 421, from amino acids 266 to 411, from amino acids 266 to 401, and from amino acids 266 to 391 in the wild-type human IL-2 receptor β chain amino acid sequence (RefSeq NP_000869.1) (SEQ ID NO: 73).

The "(iii) first intracellular region derived from an IL-2 receptor β chain" used in the present invention is preferably derived from the entire intracellular region of a native IL-2 receptor β chain, and more preferably derived from the amino acid sequence from amino acids 266 to 551 in the amino acid sequence of the wild-type human IL-2 receptor β chain (RefSeq NP_000869.1).

In the "(iii) first intracellular region derived from an IL-2 receptor β chain" used in the present invention, a mutation is introduced at one or more tyrosine residues. The "(iii) first intracellular region derived from an IL-2 receptor β chain" used in the present invention is prepared by replacing at least one amino acid in the amino acid sequence of the intracellular region derived from an IL-2 receptor β chain with another amino acid. Use of the intracellular region into which such a mutation has been introduced enables for highly efficient cellular signaling.

The position in "(iii) the first intracellular region derived from an IL-2 receptor β chain" at which the mutation is introduced is selected from the group consisting of (1) tyrosine at position 381, (2) tyrosine at position 384, and (3) tyrosine at position 387 in the amino acid sequence of the wild-type human IL-2 receptor β chain (RefSeq NP_000869.1), or is selected from the group consisting of positions in the amino acid sequence of a non-human IL-2 receptor β chain which correspond to the above-mentioned (1) to (3). The positions in the amino acid sequence of the intracellular region of a non-human IL-2 receptor β chain which correspond to the above-mentioned amino acid positions in the amino acid sequence of the intracellular region of the human IL-2 receptor β chain can be easily identified by a person skilled in the art.

In the "(iii) first intracellular region derived from an IL-2 receptor β chain" used in the present invention, the above-mentioned amino acid residue (tyrosine) is substituted with another amino acid. The amino acid residue after substitution is not particularly limited as long as the desired function of enabling highly efficient cellular signaling is obtained. Furthermore, as long as the desired function is obtained, the amino acid residue after substitution may be a natural amino acid or an artificial amino acid. Excluding a few special amino acids, there are 20 types of amino acids found in nature, which are classified into several groups based on their structures. For example, there are Group A: glycine, alanine; Group B: valine, leucine, isoleucine; Group C: aspartic acid, glutamic acid; Group D: asparagine, glutamine; Group E: serine, threonine; F group: lysine, arginine, histidine; Group G: phenylalanine, tyrosine, tryptophan; Group H: cysteine, methionine; and Group I: proline. Since amino acid residues belonging to the same group exhibit similar properties, it is expected that such amino acid residues are mutually replaceable.

The mutation introduced into the "(iii) first intracellular region derived from an IL-2 receptor β chain" is preferably a substitution of a tyrosine residue with another amino acid belonging to the above-mentioned Group G (phenylalanine, tryptophan), and more preferably a substitution of a tyrosine residue with a phenylalanine residue.

Examples of the amino acid substitution include, but not limited to, (1) substitution of tyrosine at position 381 with phenylalanine (Y381F), (2) substitution of tyrosine at position 384 with phenylalanine (Y384F), and (3) substitution of tyrosine at position 387 with phenylalanine (Y387F) in the amino acid sequence of the human IL-2 receptor β chain (RefSeq NP_000869.1) (SEQ ID NO: 73), and amino acid substitutions corresponding to the above (1) to (3) in the amino acid sequence of a non-human IL-2 receptor β chain.

Preferably, the "(iii) first intracellular region derived from an IL-2 receptor β chain" comprises one or more amino acid substitutions selected from the group consisting of the above-mentioned amino acid substitutions (1) to (3), or one or more amino acid substitutions selected from the group of amino acid substitutions corresponding to the above-mentioned (1) to (3) in the amino acid sequence of a non-human IL-2 receptor β chain.

More preferably, the "(iii) first intracellular region derived from an IL-2 receptor β chain" comprises all of the above-mentioned three amino acid substitutions (1) to (3), or all of three amino acid substitutions corresponding to the above-mentioned (1) to (3) in the amino acid sequence of a non-human IL-2 receptor β chain.

The IL-2 receptor β chain is sometimes referred to as "IL-2Rβ" or "IL2Rβ".

The "(iii) first intracellular region derived from an IL-2 receptor β chain" used in the present invention may comprise an intracellular region derived from another polypeptide in addition to the intracellular region derived from the IL-2 receptor β chain. Examples of such an intracellular region derived from another polypeptide include an intracellular region derived from a cytokine receptor, an intracellular region derived from a growth factor receptor, an intracellular region derived from a receptor tyrosine kinase, and an intracellular region derived from a receptor for a T-cell activation regulator, as well as their mutants having the same function as the above-mentioned receptors. Examples of the cytokine receptor include, but not limited to, an interleukin 1 receptor (IL-1RI), an IL-1 receptor accessory protein (IL-1RAcP), an IL-2 receptor alpha chain, an IL-2 receptor γ chain, IL-3Rα, IL-3Rβ (common β chain), IL-4Rα, IL-5Rα, IL-6Rα, gp130, IL-7Rα, IL-15Rα, IL-21R, an erythropoietin receptor (EpoR), a thrombopoietin receptor (TpoR), a granulocyte colony-stimulating factor receptor (G-CSFR), a granulocyte-macrophage colony-stimulating factor receptor (GM-CSFR), and a growth hormone receptor (GHR). Examples of the growth factor receptor include, but not limited to, a fibroblast growth factor receptor (FGFR1, FGFR2, FGFR3), an epidermal growth factor receptor (EGFR), an insulin-like growth factor 1 receptor (IGF-1R), a MET receptor, a platelet-derived growth factor receptor (PDGFR), a nerve growth factor receptor (NGFR), a TGF-β type I receptor (TβRI), and a TGF-β type II receptor (TβRII). Examples of the receptor tyrosine kinase include, but not limited to, erythropoietin-producing hepatocyte receptor A4 (EPHA4), and c-kit. Examples of the receptor for a T cell activation regulator include, but not limited to, CD27, CD28, CD40, CD40L, GITR, OX-40, ICOS, and 4-1BB. The "intracellular region derived from another polypeptide" may be added, for example, to either the N-terminus or C-terminus of the intracellular region derived from an IL-2 receptor β chain. For example, the "intracellular region derived from another polypeptide" may be fused to the N-terminus or C-terminus of the intracellular region derived from an IL-2 receptor β chain directly or via a linker. The linker may be, for example, a peptide linker, for example, a peptide linker of 2 to 10 amino acids in length.

### "(vi) Second intracellular region derived from IL-2 receptor gamma chain"

The "(vi) second intracellular region derived from an IL-2 receptor γ chain" contained in the second polypeptide constituting the chimeric receptor of the present invention may be the entire intracellular region of a native IL-2 receptor γ chain, or a partial intracellular region of a native IL-2 receptor γ chain. An example of the entire intracellular region of a native IL-2 receptor γ chain is the amino acid sequence from amino acids 284 to 369 in the wild-type human IL-2 receptor γ chain amino acid sequence (RefSeq NP_000197.1) (SEQ ID NO: 74). Examples of the partial intracellular region of a native IL-2 receptor γ chain include amino acid sequences from amino acids 284 to 359, from amino acids 284 to 349, from amino acids 284 to 339, from amino acids 284 to 329, and from amino acids 284 to 319 in the wild-type human IL-2 receptor γ chain amino acid sequence (RefSeq NP_000197.1).

The "(vi) second intracellular region derived from an IL-2 receptor γ chain" used in the present invention is preferably the entire intracellular domain of a native IL-2 receptor γ chain, and more preferably the amino acid sequence from amino acids 284 to 369 in the amino acid sequence of the wild-type human IL-2 receptor γ chain (RefSeq NP_000197.1) (SEQ ID NO: 74).

The IL-2 receptor γ chain is also called the "cytokine common gamma chain". The IL-2 receptor γ chain is sometimes referred to as "IL-2Rγ" or "IL2Rγ".

The "(vi) second intracellular region derived from an IL-2 receptor γ chain" used in the present invention may comprise an intracellular region derived from another polypeptide in addition to the intracellular region derived from the IL-2 receptor γ chain. Examples of such an intracellular region derived from another polypeptide include an intracellular region derived from a cytokine receptor, an intracellular region derived from a growth factor receptor, an intracellular region derived from a receptor tyrosine kinase, and an intracellular region derived from a receptor for a T-cell activation regulator, as well as their mutants having the same function as the above-mentioned receptors. Examples of the cytokine receptor include, but not limited to, an interleukin 1 receptor (IL-1RI), an IL-1 receptor accessory protein (IL-1RAcP), an IL-2 receptor alpha chain, an IL-2 receptor β chain, IL-3Rα, IL-3Rβ (common β chain), IL-4Rα, IL-5Rα, IL-6Rα, gp130, IL-7Rα, IL-15Rα, IL-21R, an erythropoietin receptor (EpoR), a thrombopoietin receptor (TpoR), a granulocyte colony-stimulating factor receptor (G-CSFR), a granulocyte-macrophage colony-stimulating factor receptor (GM-CSFR), and a growth hormone receptor (GHR). Examples of the growth factor receptor include, but not limited to, a fibroblast growth factor receptor (FGFR1, FGFR2, FGFR3), an epidermal growth factor receptor (EGFR), an insulin-like growth factor 1 receptor (IGF-1R), a MET receptor, a platelet-derived growth factor receptor (PDGFR), a nerve growth factor receptor (NGFR), a TGF-β type I receptor (TβRI), and a TGF-β type II receptor (TβRII). Examples of the receptor tyrosine kinase include, but not limited to, erythropoietin-producing hepatocyte receptor A4 (EPHA4), and c-kit. Examples of the receptor for a T cell activation regulator include, but not limited to, CD27, CD28, CD40, CD40L, GITR, OX-40, ICOS, and 4-1BB. The "intracellular region derived from another polypeptide" may be added, for example, to either the N-terminus or C-terminus of the intracellular region derived from an IL-2 receptor γ chain, and is preferably added to the C-terminus. For example, the "intracellular region derived from another polypeptide" may be fused to the N-terminus or C-terminus of the intracellular region derived from an IL-2 receptor γ chain directly or via a linker. The linker may be, for example, a peptide linker, for example, a peptide linker of 2 to 10 amino acids in length.

The "intracellular region derived from another polypeptide" used in the present invention is preferably an intracellular region of CD40, and more preferably the amino acid sequence from amino acids 216 to 277 (SEQ ID NO: 71) in the amino acid sequence of wild-type human CD40 (RefSeq NP_001241.1).

In a preferred embodiment of the present invention, the first polypeptide is, for example, a polypeptide comprising amino acids 65 to 601 of an amino acid sequence of SEQ ID NO: 3 in which one or more tyrosines selected from the group consisting of tyrosines at positions 431, 434 and 437 are substituted with other amino acid. In a preferred embodiment of the present invention, the second polypeptide is, for example, a polypeptide comprising amino acids 64 to 396 of the amino acid sequence of SEQ ID NO: 1.

The present invention provides a precursor of the chimeric receptor. The precursor is characterized by comprising a first polypeptide and a second polypeptide in which they are connected to each other via a self-cleaving peptide. More specifically, the precursor of the chimeric receptor of the present invention is characterized by comprising a first polypeptide and a second polypeptide in which they are connected to each other via a self-cleaving peptide, the first polypeptide comprising (i) a first extracellular region that binds to a ligand, (ii) a first transmembrane region, and (iii) a first intracellular region derived from an IL-2 receptor β chain, wherein a mutation is introduced at one or more tyrosine residues in the first intracellular region; and the second polypeptide comprising (iv) a second extracellular region that binds to a ligand, (v) a second transmembrane region, and (vi) a second intracellular region derived from an IL-2 receptor γ chain. Here, arrangement of the first and second polypeptides is not particularly limited, and may be either "the first polypeptide - the self-cleaving peptide - the second polypeptide" or "the second polypeptide - the self-cleaving peptide - the first polypeptide". Further, a linker peptide may be added to one or both ends of the self-cleaving peptide.

As used herein, the term "self-cleaving peptide" means a peptide sequence with activity of cleavage that occurs between two amino acid residues within the peptide sequence itself. Self-cleaving peptides are peptides of 18-22 amino acids in length that induce ribosomal skipping during protein translation in cells. Self-cleaving peptides are found in various viral families and share a consensus motif (DxExNPGP). Self-cleaving peptides inhibit the transferase activity of ribosomes, preventing peptide bond formation. Thus, when a self-cleaving peptide is placed between the upstream and downstream genes, translation is disrupted, and as a result, the co-expression of the two genes becomes possible.

For example, in 2A peptides and 2A-like peptides, cleavage occurs between a glycine residue and a proline residue on these peptides. This occurs due to a "ribosome skipping mechanism" in which the normal peptide bond formation between the glycine residue and the proline residue does not occur during translation, without affecting downstream translation. The ribosomal skipping mechanism is known in the art, and is used for the expression of multiple proteins encoded by a single messenger RNA (mRNA) molecule.

Examples of the self-cleaving peptide used in the present invention include a P2A peptide derived from Porcine teschovirus (PTV), T2A derived from Thosea asigna virus (TaV), F2A derived from foot and mouth disease virus (FMDV), and E2A derived from equine rhinitis A virus (ERAV).

### (2) Nucleic acid of the present invention

The present invention provides a nucleic acid encoding the chimeric receptor of the present invention as described above in (1). That is, the nucleic acid of the present invention encodes a chimeric receptor comprising a first polypeptide and a second polypeptide, the first polypeptide comprising (i) a first extracellular region that binds to a ligand, (ii) a first transmembrane region, and (iii) a first intracellular region derived from an IL-2 receptor β chain, wherein a mutation is introduced at one or more tyrosine residues in the first intracellular region; and the second polypeptide comprising (iv) a second extracellular region that binds to a ligand, (v) a second transmembrane region, and (vi) a second intracellular region derived from an IL-2 receptor γ chain.

In a preferred embodiment of the present invention, a nucleic acid encoding the first polypeptide is exemplified by a nucleic acid of a nucleotide sequence encoding a polypeptide comprising amino acids 65 to 601 of an amino acid sequence of SEQ ID NO: 3 in which one or more tyrosines selected from the group consisting of tyrosines at positions 431, 434 and 437 are substituted with other amino acids. In a preferred embodiment of the present invention, a nucleic acid encoding the second polypeptide is exemplified by a nucleic acid of a nucleotide sequence encoding a polypeptide comprising amino acids 64 to 396 of the amino acid sequence of SEQ ID NO:1.

The present invention also provides a nucleic acid encoding the precursor of the chimeric receptor.

The nucleic acid of the present invention is capable of expressing the chimeric receptor of the present invention in a cell. Since the cell expressing the chimeric receptor of the present invention acquires the ability to proliferate in a ligand-dependent manner, the cell can be specifically proliferated, i.e., the proliferation of the cell can be selectively controlled. Thus, the nucleic acid of the present invention is referred to as a "selective regulatory gene (SRG)".

The type of nucleic acid used in the present invention is not limited, and a single-stranded nucleic acid, a double-stranded nucleic acid, DNA, and RNA can be used. Examples of DNA include double-stranded DNA, single-stranded DNA, and cDNA. Examples of RNA include mRNA.

The nucleic acid of the present invention may be linked to a nucleic acid comprising a promoter sequence so as to be expressed in a cell. Examples of the promoter include a promoter that constitutively promotes the expression of a gene or an operably linked construct, and a promoter that induces the expression of a gene or an operably linked construct by the action of a drug, etc. (e.g., tetracycline or doxorubicin). For efficient transcription, the nucleic acid of the present invention may be also linked to a nucleic acid comprising other regulatory elements that cooperate with a promoter or transcription initiation site, such as an enhancer sequence or a terminator sequence.

In a certain embodiment, the nucleic acid of the present invention is codon-optimized for expression in a particular host cell.

### (3) Vector of the present invention

The present invention provides a vector containing the nucleic acid of the present invention as described above in (2). That is, the vector of the present invention contains a nucleic acid encoding a chimeric receptor comprising a first polypeptide and a second polypeptide, the first polypeptide comprising (i) a first extracellular region that binds to a ligand, (ii) a first transmembrane region, and (iii) a first intracellular region derived from an IL-2 receptor β chain, wherein a mutation is introduced at one or more tyrosine residues in the first intracellular region; and the second polypeptide comprising (iv) a second extracellular region that binds to a ligand, (v) a second transmembrane region, and (vi) a second intracellular region derived from an IL-2 receptor γ chain.

The vector of the present invention is capable of expressing the first polypeptide and the second polypeptide in a desired cell.

Embodiments of the vector of the present invention are exemplified by (a) a vector containing both a nucleic acid encoding the first polypeptide and a nucleic acid encoding the second polypeptide, and (b) a combination of a vector containing a nucleic acid encoding the first polypeptide and a vector containing a nucleic acid encoding the second polypeptide. In the above embodiment (a), the nucleic acid encoding the first polypeptide and the nucleic acid encoding the second polypeptide may be transcribed and translated by different promoters, or may be transcribed and translated by a single promoter by using an internal ribosome entry site (IRES). Furthermore, a nucleic acid encoding a polypeptide formed by connecting the first polypeptide and the second polypeptide to each other via a self-cleaving peptide may be transcribed and translated by a single promoter. In the above embodiment (b), the two vectors may be the same type of vector or different types of vectors.

The vectors that can be used in the present invention are operably linked to a suitable control sequence so as to express the nucleic acid of the present invention in a suitable host cell. Examples of the control sequence include a promoter for transcribing the nucleic acid of the present invention, an optional operator sequence for controlling transcription, a sequence encoding a ribosome binding site, an enhancer, a polyadenylation sequence, and a sequence controlling the termination of transcription and translation. Examples of a promoter for controlling transcription in a mammalian cell include mammalian-derived promoters (PGK promoter, EF1-α promoter, β-globin promoter, etc.), virus-derived promoters (CMV promoter, SV40 promoter, MMLV-LTR promoter, HIV-LTR promoter, etc.), and artificially constructed promoters (CAG promoter, etc.). Furthermore, the vector may contain various sequences known to a person skilled in the art, for example, a restriction enzyme cleavage site, a marker gene (selection gene) such as a drug resistance gene, a signal sequence, a leader sequence, or the like, as necessary. These various sequences or sites can be appropriately selected and used by a person skilled in the art depending on the type of a polypeptide to be expressed, a host cell to be used, a culture medium, and other conditions.

The vector of the present invention may contain, in addition to the nucleic acid of the present invention, a gene that can serve as a marker for confirming that the vector has been introduced into a cell (e.g., a drug resistance gene, a gene encoding a reporter enzyme, or a gene encoding a fluorescent protein) or a therapeutic gene.

The therapeutic gene used in the present invention is not particularly limited as long as it exerts a favorable effect on a disease for which treatment or prevention is desired. Examples of the therapeutic gene include a polypeptide, a ribozyme, antisense RNA, and RNA exhibiting RNA interference activity. Since the present invention brings about the expression of a therapeutic gene in response to the onset of a disease, for example carcinogenesis or viral infection, a gene useful for eliminating cancer cells or virally infected cells, for example a gene that can exert cytotoxicity, can be used. Examples of the therapeutic gene include, but not limited to, a gene encoding a CAR, a gene encoding a TCR, a gene encoding a polypeptide having protease activity, a gene encoding a polypeptide having nuclease activity, a gene encoding a cytokine, and a gene involved in nucleic acid metabolism.

Preferably, the vector of the present invention further contains a nucleic acid encoding a CAR or a foreign TCR that recognizes a target cell in disease treatment.

CAR refers to a fusion protein comprising an extracellular domain that binds to an antigen (hereinafter, referred to as an antigen-binding domain), a transmembrane domain derived from a polypeptide different from the antigen-binding domain, and at least one intracellular domain. CAR is sometimes called a "chimeric antigen receptor", a "chimeric receptor", a "T-body", or a "chimeric immune receptor (CIR)".

The "antigen-binding domain" means a portion capable of binding to an antigen. A portion of an antibody which has the antigen-binding ability can be used as the antigen-binding domain. Examples of the antigen-binding domain include a Fab' fragment, a Fab fragment, and an Fv fragment. A single chain variable fragment (scFv) is preferably used in the present invention. The scFv means a single-chain polypeptide derived from an antibody which retains the antigen-binding ability. An example of scFv is a polypeptide formed by connecting an Fv region of an immunoglobulin heavy chain (H chain) and an Fv region of an immunoglobulin light chain (L chain) via a spacer sequence by recombinant DNA techniques. Various methods for producing scFv are known, and examples thereof include methods described in U.S. Pat. No. 4694778; Science, vol. 242, pp. 423-442 (1988); Nature, vol. 334, p. 54454 (1989); and Science, vol. 242, pp. 1038-1041 (1988). The antigen-binding domain is not limited to a polypeptide derived from an antibody. A polypeptide derived from a protein other than an antibody can also be used as the antigen-binding domain. For example, the full-length or a part of a protein such as APRIL or GM-CSF can be used as the antigen-binding domain.

The "intracellular domain" means any oligopeptide or polypeptide known to function as a domain that transmits a signal that leads to the activation or inhibition of a biological process within a cell.

A typical CAR structure consists of an scFv, a transmembrane domain, and an intracellular domain that activates cells. Known examples of the transmembrane domain include transmembrane domains derived from TCR complexes CD3ζ, CD28, CD8α, and the like. A preferable example of the intracellular domain is the intracellular domain of TCR complex CD3ζ. A CAR having such a configuration is called the first-generation CAR. T cells expressing CAR (CAR-T cells) directly recognize surface antigens of tumor cells regardless of the expression of major histocompatibility complex class I on the tumor cells, while the T cells themselves are activated. Thus, the T cells expressing CAR can efficiently kill tumor cells.

For the purpose of enhancing the T cell activation ability of the first-generation CAR, the second-generation CAR in which the intracellular domain of a T cell costimulatory molecule is linked has been developed. Preferable examples of the T cell costimulatory molecule include the intracellular domain of CD28, the intracellular domains of CD137 (4-1BB) and CD134 (OX40) which are members of the tumor necrosis factor (TNF) receptor superfamily, the intracellular domain of an interleukin receptor and modified products thereof, and the intracellular domain of a glucocorticoid-induced tumor necrosis factor receptor (GITR). As a further improved type, the third-generation CAR in which the intracellular domains of these costimulatory molecules are linked in tandem has been developed, and many CAR molecules targeting various tumor antigens have been reported. The nucleic acid construct of the present invention may contain a sequence encoding any CAR as the sequence of a desired gene.

As used herein, the term TCR refers to a molecule responsible for the antigen recognition function of T cells, and is composed of polypeptides such as an α chain, a β chain, a γ chain, and a δ chain. Of these, a heterodimer of the TCR alpha chain (TCR alpha) and the TCR beta chain (TCR beta), or a heterodimer of the gamma chain and the delta chain, together with accessory molecules such as a CD3 complex (including gamma, delta, epsilon, and zeta), CD4, or CD8, forms the TCR.

In the body, the TCR recognizes peptides (antigen epitopes) presented by target cells (phagocytes, virus-infected cells, cancer cells, etc.) via the major histocompatibility complex (MHC) and induces an immune response against the target cells. The human MHC is also called the human histocompatibility leukocyte antigen (HLA) system. HLA is classified into class I and class II; class I including HLA-A, B, C, E, F, G, H and J, and class II including HLA-DR, DQ and DP. Like TCR, HLA is formed from a complex of α and β chains, and presents antigen epitopes on the cell surface via these chains. HLA class I is present on almost all cells of the body and presents antigen epitopes of approximately 9 amino acid residues in length. For example, when a virus-infected cell presents an antigen epitope derived from the virus via HLA, a T cell (e.g., a cytotoxic T lymphocyte) having a TCR specific to the antigen epitope is activated, resulting in the body's immune response (e.g., killing of the virus-infected cell) and thus providing biological defense.

The TCR usually induces an immune response only when binding to a complex formed by a particular type of HLA and an antigen epitope. This restriction is called "HLA restriction". As used herein, the term HLA (or MHC) restriction may be used in conjunction with various peptides, cells, etc. in the context of a TCR, a T cell expressing such a TCR, or a presented antigen-HLA complex. The binding of a TCR to an antigen epitope-HLA complex results in an immune response, and an example of such an immune response is the secretion of cytokines (interferon-γ, TNF-α, IL-2, etc.) by CD8-positive T cells.

The gene that can serve as a marker or the therapeutic gene may be contained in the vector in such a manner as to be expressed by a promoter different from that of the nucleic acid encoding the chimeric receptor of the present invention, or by the same promoter as that of the nucleic acid encoding the chimeric receptor of the present invention. Furthermore, a nucleic acid encoding a fusion polypeptide formed by connecting a precursor of the chimeric receptor of the present invention and a polypeptide encoded by the gene that can serve as a marker and/or the therapeutic gene via a self-cleaving peptide may be connected to a promoter and carried in the vector.

The vector that can be used in the present invention include a vector that is integrated into the genome of a host cell, a vector that is not integrated into the genome of a host cell, and an episomal vector that exists in the cytoplasm and replicates autonomously. For example, a plasmid vector, a viral vector, or an artificial chromosome can be suitably used. Examples of the viral vector that can be used include retroviral vectors (including oncoretroviral vectors, lentiviral vectors, and pseudotype vectors), adenoviral vectors, adeno-associated virus (AAV) vectors, simian virus vectors, vaccinia virus vectors, Sendai virus vectors, Epstein-Barr virus (EBV) vectors, and HSV vectors. The viral vector may preferably be replication-defective so that the virus cannot replicate autonomously in cells infected with the virus. Various viral vectors and methods for their production are well known to a person skilled in the art. Commercially available viral vectors may also be used in the present invention.

Non-viral vectors can also be used in combination with condensing agents such as liposomes and cationic lipids. Furthermore, the nucleic acid of the present invention can be introduced into cells by calcium phosphate transfection, DEAE-dextran, electroporation, or particle bombardment.

### (4) Cells of the present invention

The present invention provides a cell expressing the chimeric receptor of the present invention as described above in (1). That is, the cell of the present invention expresses a chimeric receptor comprising a first polypeptide and a second polypeptide, the first polypeptide comprising (i) a first extracellular region that binds to a ligand, (ii) a first transmembrane region, and (iii) a first intracellular region derived from an IL-2 receptor β chain, wherein a mutation is introduced at one or more tyrosine residues in the first intracellular region; and the second polypeptide comprising (iv) a second extracellular region that binds to a ligand, (v) a second transmembrane region, and (vi) a second intracellular region derived from an IL-2 receptor γ chain.

The cell of the present invention has the ability to specifically proliferate upon stimulation with a ligand. That is, when the cell *in vitro* or *in vivo* comes into contact with a ligand that binds to the chimeric receptor expressed by the cell, proliferation within the cell is initiated.

The origin of the cell of the present invention is not particularly limited, and a cell derived from a mammal, for example, a human cell, or a cell derived from a non-human mammal such as monkey, mouse, rat, pig, horse, or dog can be used. The cell of the present invention is preferably a human cell.

The type of cell of the present invention is not particularly limited, and any type of cell can be used. For example, cells harvested, isolated or purified from body fluids, tissues or organs, such as blood (peripheral blood, umbilical cord blood, etc.) or bone marrow, or cells obtained by differentiating the above-mentioned cells or reprogramming the above-mentioned cells to generate pluripotent stem cells (iPS cells) can be used (see, for example, Themeli et al. 2013). Examples of the cell that can be used include peripheral blood mononuclear cells (PBMC), immune cells, umbilical cord blood mononuclear cells, fibroblasts, adipocyte precursors, hepatocytes, skin keratinocytes, mesenchymal stem cells, adipose stem cells, various cancer cell lines, and neural stem cells. Examples of the cell that can be used include NK cells and T cells, precursor cells of T cells (hematopoietic stem cells, lymphocyte precursor cells, etc.), and cell populations containing the above-mentioned cells. Examples of T cells include CD8 positive T cells, CD4 positive T cells, regulatory T cells, cytotoxic T cells, and tumor infiltrating lymphocytes. The cell population containing T cells and T cell precursors include a PBMC. The above-mentioned cells may be obtained from a living organism, may be obtained by expansion of a cell obtained from a living organism, or may be established as a cell line. Furthermore, a cell obtained by differentiation from a pluripotent stem cell (ES cell, iPS cell, etc.), for example an immune cell, may be used as the cell of the present invention. The iPS cell produced from a T cell clone having the ability to recognize and damage a specific cell can be differentiated to obtain a T cell. The T cell thus obtained has the same specific cytotoxic activity as the starting T cell clone and is in a more undifferentiated state. Into the T cell, the nucleic acid encoding the chimeric receptor of the present invention is introduced, thereby a cell that exerts excellent therapeutic effect can be easily prepared.

As used herein, the term "immune cell" refers to a general cell involved in immune function in the body, and examples of such a cell include a hematopoietic stem cell, a neutrophil, a basophil, a macrophage, a lymphocyte (B cell and T cell), a monocyte, a dendritic cell, a natural killer (NK) cell, and a plasma cell. Of these, a T cell and an NK cell are preferred for the present invention. As used herein, the "immune cell" also includes "a precursor cell of an immune cell" that has the ability to differentiate into the above-mentioned immune cell.

When transplantation of the cell of the present invention or a cell differentiated from the cell of the present invention into an organism is desired, it is preferable to introduce the nucleic acid into a cell taken from the organism itself to be transplanted or a same kind of organism.

The cell of the present invention may further express a polypeptide (enzyme, antibody, cytokine, etc.) or a nucleic acid (siRNA, etc.) useful for treating a disease, preferably a CAR or a foreign TCR that recognizes a target cell.

As used herein, the term "target cell" refers to a cell desired to be reduced or eliminated in a patient, and examples of such a cell include a tumor cell and a cell infected with a pathogen. The target cell is preferably a tumor cell, and more preferably a hematopoietic tumor cell or a solid tumor cell.

The cell of the present invention that further expresses a CAR or a foreign TCR that recognizes a target cell has the ability to recognize a desired target cell and destroys the target cell by its cytotoxic activity. The cytotoxic activity can be evaluated by a known method. For example, the cytotoxic activity of the cell of the present invention against the target cell labeled with a radioactive substance, a fluorescent substance or the like can be evaluated by measuring the radioactivity or fluorescence intensity from the target cell destroyed by the cell of the present invention. The cytotoxic activity can be also detected by measuring the amount of cytokine such as GM-CSF or IFN-γ that is specifically released from the cell of the present invention or the target cell.

### (5) Production method for cell of the present invention

The present invention provides a method for producing a cell, the method comprising a step of introducing the nucleic acid of the present invention as described above in (2) into a cell. That is, the method for producing the cell of the present invention comprises a step of introducing into a cell a nucleic acid encoding a chimeric receptor comprising a first polypeptide and a second polypeptide, the first polypeptide comprising (i) a first extracellular region that binds to a ligand, (ii) a first transmembrane region, and (iii) a first intracellular region derived from an IL-2 receptor β chain, wherein a mutation is introduced at one or more tyrosine residues in the first intracellular region; and the second polypeptide comprising (iv) a second extracellular region that binds to a ligand, (v) a second transmembrane region, and (vi) a second intracellular region derived from an IL-2 receptor γ chain.

The step is carried out ex vivo. For example, the cell can be produced by transducing a cell ex vivo using a viral or non-viral vector containing the nucleic acid of the present invention.

For the cell production method of the present invention, a cell derived from a mammal, for example, a human cell, or a cell derived from a non-human mammal such as monkey, mouse, rat, pig, cow, or dog can be used. The cell used in the method of the present invention is not particularly limited, and any of the above-mentioned cells can be used. The cell may be a cell taken from a living body, a cell obtained by expansion of a cell taken from a living body, or an established cell line. When the produced cell or a cell differentiated from the produced cell is desired to be transplanted into an organism, it is preferable to introduce the nucleic acid into a cell taken from the organism itself to be transplanted or a same kind of organism. Furthermore, cells before the introduction of nucleic acid may be stimulated to increase the introduction efficiency of the nucleic acid into the cells, or to enrich a specific type of cells. For example, when a PBMC is cultured in the presence of an anti-CD3 antibody, the proportion of T cells is increased. When a PBMC is cultured in the presence of both an anti-CD3 antibody and RetroNectin which is a recombinant fibronectin fragment, a cell population rich in CD8 positive T cells is obtained.

The nucleic acid encoding the chimeric receptor of the present invention is inserted into a vector, and the vector can be introduced into a cell. As the vector, the vector of the present invention described above in (3) can be used. A method for introducing the vector into a cell is not particularly limited, and an appropriate method may be selected depending on the vector and cell type used.

A non-viral vector can also be used in combination with an adjuvant such as liposome, cationic lipid, or polyethylene imine, in the present invention. Furthermore, the nucleic acid of the present invention can be introduced into a cell by calcium phosphate transfection, DEAE-dextran, electroporation, or particle bombardment.

Methods for producing various viral vectors and methods for introducing them into cells are also well known to a person skilled in the art. For example, for a retroviral vector, an appropriate packaging cell is selected depending on the LTR sequence and packaging signal sequence contained in the vector, and the packaging cell can be used to prepare retroviral particles. Examples of the packaging cell include PG13 (ATCC CRL-10686), PA317 (ATCC CRL-9078), GP+E-86 and GP+envAm-12 (U.S. Patent No. 5278056), and Psi-Crip [Proceedings of the National Academy of Sciences, Vol. 85, pp. 6460-6464 (1988)]. Retroviral particles can also be produced using 293 cells or 293T cells, which have high transfection efficiency. Retroviral vectors produced based on many types of retroviruses and packaging cells that can be used for packaging of the vectors are widely available commercially from various companies.

The cell into which the nucleic acid of the present invention has been introduced can be cultured *ex vivo* to expand the cell number. For culturing the cell, a medium suitable for the cell may be used, and such a medium may be selected from known or commercially available media. For example, media that can be used for culturing lymphocytes and other immune cells are known to a person skilled in the art, and media of various compositions are commercially available. There are media containing a human- or animal-derived serum (e.g., fetal bovine serum; FBS or FCS), xeno-free media, and defined media which do not contain unknown components, etc., and such media can be selected according to the purpose. For cell culture, a known culture vessel (plate, petri dish, flask, bag, culture tank, etc.) can be used. The culture conditions may be the same as those for normal cell culture (e.g., 32-37°C, 5% CO₂, etc.). Furthermore, at appropriate time intervals, the cell culture may be diluted by adding a fresh medium, or the medium or the culture vessel may be exchanged.

In the production method for the cell of the present invention, the proliferation of the cell of the present invention can be promoted by culturing the cell in contact with the ligand to which the chimeric receptor binds. When a cell population containing the cell of the present invention and other cells is cultured in a medium containing the ligand, the cell of the present invention receives a proliferation signal by the binding to the ligand and actively proliferates. On the other hand, the other cells grow at a normal rate. Thus, a cell population enriched in the cell of the present invention can be obtained. The concentration of the ligand may be appropriately adjusted depending on the types of ligand and cell. For example, in the case of the cell expressing the chimeric receptor comprising an extracellular region derived from an erythropoietin receptor, the cell proliferation is promoted in a medium containing 0.9 IU/mL or more of erythropoietin in an erythropoietin concentration-dependent manner. Thus, the cell of the present invention can be selectively produced. Therefore, the desired highly pure cell population can be obtained without any purification procedure or with only a simple purification procedure. Furthermore, the cell obtained by the production method for the cell of the present invention is characterized by having a higher antitumor effect than the control.

### (6) Pharmaceutical composition of the present invention

The present invention provides a pharmaceutical composition comprising the cell of the present invention as described above in (4) as an active ingredient. That is, the pharmaceutical composition of the present invention comprises, as an active ingredient, a cell expressing a chimeric receptor comprising a first polypeptide and a second polypeptide, the first polypeptide comprising (i) a first extracellular region that binds to a ligand, (ii) a first transmembrane region, and (iii) a first intracellular region derived from an IL-2 receptor β chain, wherein a mutation is introduced at one or more tyrosine residues in the first intracellular region; and the second polypeptide comprising (iv) a second extracellular region that binds to a ligand, (v) a second transmembrane region, and (vi) a second intracellular region derived from an IL-2 receptor γ chain.

The pharmaceutical composition of the present invention may comprise a pharmaceutically acceptable excipient. Pharmaceutically acceptable excipients are well known to a person skilled in the art, and examples thereof include phosphate buffered saline (e.g., 0.01 M phosphate, 0.138 M NaCl, 0.0027 M KCl, pH 7.4), aqueous solutions containing mineral acid salts such as hydrochloride, hydrobromide, phosphate, sulfate, etc., saline, solutions of glycol or ethanol, etc., and salts of organic acids such as acetate, propionate, malonate, benzoate, etc. Auxiliary substances such as a wetting agent and an emulsifying agent, and a pH buffering agent can also be used. Pharmaceutically acceptable excipients are described in Remington's Pharmaceutical Sciences (Mack Pub. Co., N.J. 1991). The pharmaceutical composition can be in any known form suitable for parenteral administration, for example injection or infusion. Furthermore, formulation additives, for example a suspending agent, a preserving agent, a stabilizer and/or a dispersing agent, and/or a preservative for extending a shelf life during storage may be used.

### (7) Method for preventing or treating disease of the present invention

The present invention provides a method for preventing or treating a disease, the method comprising administering the cell of the present invention as described above in (4) or the pharmaceutical composition of the present invention as described above in (6) to a subject. That is, the method for preventing or treating a disease of the present invention comprises administering, to a subject, a cell expressing a chimeric receptor comprising a first polypeptide and a second polypeptide, the first polypeptide comprising (i) a first extracellular region that binds to a ligand, (ii) a first transmembrane region, and (iii) a first intracellular region derived from an IL-2 receptor β chain, wherein a mutation is introduced at one or more tyrosine residues in the first intracellular region; and the second polypeptide comprising (iv) a second extracellular region that binds to a ligand, (v) a second transmembrane region, and (vi) a second intracellular region derived from an IL-2 receptor γ chain; or a pharmaceutical composition containing the cell as an active ingredient.

The disease is not particularly limited as long as the disease is sensitive to the cell. Examples of the disease include, but not limited to, cancer [blood cancer (leukemia, lymphoma, myeloma), solid tumors, etc.], inflammatory diseases/autoimmune diseases (asthma, eczema), hepatitis, and infectious diseases caused by viruses such as influenza, HIV and others, bacteria, and fungi, such as tuberculosis, MRSA, VRE, and deep mycoses. For example, the cell of the present invention is administered for the treatment of the disease, wherein the cell of the present invention has the ability to bind to an antigen, i.e., a tumor antigen, a viral antigen, a bacterial antigen, etc., possessed by a cell whose reduction or elimination is desired in the disease. For example, the cell of the present invention expressing a CAR specific to a tumor antigen, or the cell of the present invention expressing a foreign TCR that recognizes a tumor cell is extremely useful for tumor treatment. Furthermore, an NK cell or a cytotoxic T lymphocyte (CTL) clone expressing the chimeric receptor of the present invention can also be used to treat the disease for which the cell exerts a therapeutic effect.

The cell or the pharmaceutical composition can be administered intradermally, subcutaneously, intravenously, or the like, and may be administered systemically or locally to an affected area or a tissue in the vicinity thereof. For example, the cell or the pharmaceutical composition may be administered locally, subcutaneously. A dose can be adjusted appropriately depending on the age and weight of the subject to be administered, administration method, etc. For this purpose, the number of doses and an administration interval for multiple doses are also be taken into consideration.

In the method for preventing or treating a disease of the present invention, the ligand that binds to the chimeric receptor expressed by the cell of the present invention is administered to a subject to which the cell of the present invention has been administered, thereby the in *vivo* proliferation of the cell of the present invention can be induced, and/or the *in vivo* survival period of the cell of the present invention can be extended. A method of administering the ligand is not particularly limited, and an appropriate method is selected taking into consideration distribution of the cells of the present invention in the body, etc. A dose and an administration schedule of the ligand may be appropriately determined taking into consideration the types and doses of cells to be combined, the type of disease, and the like.

### EXAMPLES

Hereinafter, the present invention will be explained in more detail with reference to Examples, which the scope of the present invention is not limited to.

### Example 1. Preparation of SRG construct

### (1) pEX-A2J2/TEG

A nucleic acid encoding a seamless fusion polypeptide (T2A-EPOR-IL2Rγ) of a T2A sequence, a polypeptide from positions 2 to 250 (including a signal sequence and an extracellular domain) of the human erythropoietin receptor amino acid sequence shown by NCBI Reference Sequence: NP_000112.1, and a polypeptide from positions 263 to 369 (including a transmembrane domain and an intracellular domain) of the human IL-2 receptor γ chain (common γ chain) amino acid sequence shown by NCBI Reference Sequence: NP_000197.1 was prepared. This was named TEG. The amino acid sequence of this fusion polypeptide is shown in SEQ ID NO: 1. In the amino acid sequence of SEQ ID NO: 1, positions 1 to 40 correspond to a self-cleaving peptide sequence (furin-spacer-T2A), positions 41 to 63 correspond to the signal peptide sequence of the erythropoietin receptor, positions 64 to 289 correspond to the extracellular domain of the erythropoietin receptor, positions 290 to 310 correspond to the transmembrane domain of the IL-2 receptor γ chain, and positions 311 to 396 correspond to the intracellular domain of the IL-2 receptor γ chain. The nucleotide sequence of the nucleic acid is shown in SEQ ID NO: 2. The nucleic acid was synthesized as an artificial gene (T2A and EPOR were codon-optimized) and cloned into a pEX-A2J2 vector (manufactured by Eurofins Genomics) to produce plasmid pEX-A2J2/TEG.

### (2) pEX-A2J2/PEB

A nucleic acid encoding a seamless fusion polypeptide (P2A-EPOR-IL2Rβ) of a P2A sequence, a polypeptide from positions 2 to 250 (including a signal sequence and an extracellular domain) of the human erythropoietin receptor amino acid sequence shown by NCBI Reference Sequence: NP_000112.1, and a polypeptide from positions 241 to 551 (including a transmembrane domain and an intracellular domain) of the human IL-2 receptor β chain amino acid sequence shown by NCBI Reference Sequence: NP_000869.1 was prepared. This was named PEB. The amino acid sequence of this fusion polypeptide is shown in SEQ ID NO: 3. In the amino acid sequence of SEQ ID NO: 3, positions 1 to 41 correspond to a self-cleaving peptide sequence (furin-spacer-P2A), positions 42 to 64 correspond to the signal peptide sequence of the erythropoietin receptor, positions 65 to 290 correspond to the extracellular domain of the erythropoietin receptor, positions 291 to 315 correspond to the transmembrane domain of the IL-2 receptor β chain, and positions 316 to 601 correspond to the intracellular domain of the IL-2 receptor β chain. The nucleotide sequence of the nucleic acid is shown in SEQ ID NO: 4. The nucleic acid was synthesized as an artificial gene (P2A and EPOR were codon-optimized) and cloned into a pEX-A2J2 vector to prepare plasmid pEX-A2J2/PEB.

### (3) pEX-A2J2/TEGPEB

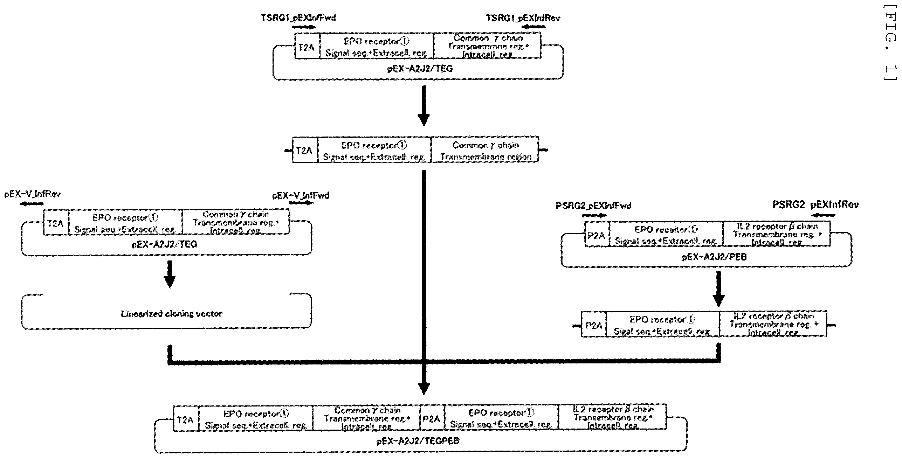

A nucleic acid encoding a seamless fusion polypeptide (T2A-EPOR-IL2Rγ-P2A-EPOR-IL2Rβ) of the fusion polypeptide (SEQ ID NO: 1) encoded by TEG and the fusion polypeptide (SEQ ID NO: 3) encoded by PEB was prepared. This was named TEGPEB. The amino acid sequence of this fusion polypeptide is shown in SEQ ID NO: 5. The nucleotide sequence of the nucleic acid is shown in SEQ ID NO: 6. As shown in FIG. 1, PCR was performed using pEX-A2J2/TEG as a template, a pEX-V_InfRev primer (SEQ ID NO: 7) and a pEX-V_InfFwd primer (SEQ ID NO: 8) to prepare a linearized vector for cloning. Next, PCR was performed using pEX-A2J2/TEG as a template, a TSRG1_pEXInfFwd primer (SEQ ID NO: 9) and a TSRG1_pEXInfRev primer (SEQ ID NO: 10) to obtain an amplified fragment containing a DNA encoding T2A-EPOR-IL2Rγ. Similarly, PCR was performed using pEX-A2J2/PEB as a template, a PSRG2_pEXInfFwd primer (SEQ ID NO: 11) and a PSRG2_pEXInfRev primer (SEQ ID NO: 12) to obtain an amplified fragment containing a DNA encoding P2A-EPOR-IL2Rβ. These two amplification products were cloned into the linearized vector for cloning as described above using In-Fusion HD Cloning Kit (manufactured by TAKARA BIO INC.) to prepare plasmid pEX-A2J2/TEGPEB.

### (4) pMEI-5/ZG-SRG_YYY

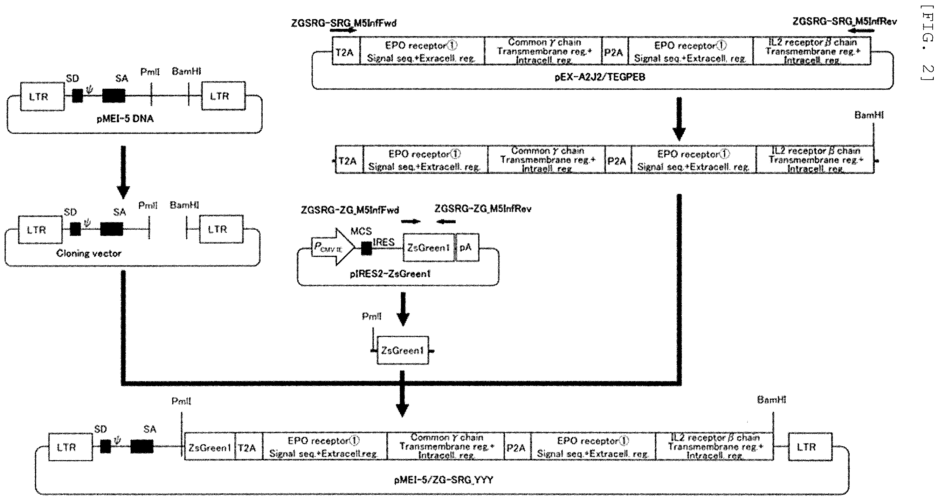

A nucleic acid encoding a seamless fusion polypeptide (ZsGreen1-T2A-EPOR-IL2Rγ-P2A-EPOR-IL2Rβ) of ZsGreen1 and the fusion polypeptide (SEQ ID NO: 5) encoded by TEGPEB was prepared. This was named ZG-SRG_YYY. The amino acid sequence of this fusion polypeptide is shown in SEQ ID NO: 13. The nucleotide sequence of the nucleic acid is shown in SEQ ID NO: 14. As shown in FIG. 2, a pMEI-5 DNA vector (manufactured by TAKARA BIO INC.) was digested with restriction enzymes PmlI and BamHI to prepare a linearized vector for cloning. Next, PCR was performed using a pIRES2-ZsGreen1 vector (manufactured by TAKARA BIO INC.) as a template, a ZGSRG-ZG_M5InfFwd primer (SEQ ID NO: 15) and a ZGSRG-ZG_M5InfRev primer (SEQ ID NO: 16) to obtain an amplified fragment containing a DNA encoding ZsGreen1. Similarly, PCR was performed using pEX-A2J2/TEGPEB as a template, a ZGSRG-SRG_M5InfFwd primer (SEQ ID NO: 17) and a ZGSRG-SRG_M5InfRev primer (SEQ ID NO: 18) to obtain an amplified fragment containing a DNA encoding T2A-EPOR-IL2Rγ-P2A-EPOR-IL2Rβ. These two amplification products were cloned into the linearized vector for cloning as described above using the In-Fusion HD Cloning Kit to prepare retroviral plasmid pMEI-5/ZG-SRG_YYY.

### (5) pMEI-S/ZG-SRG_FYY

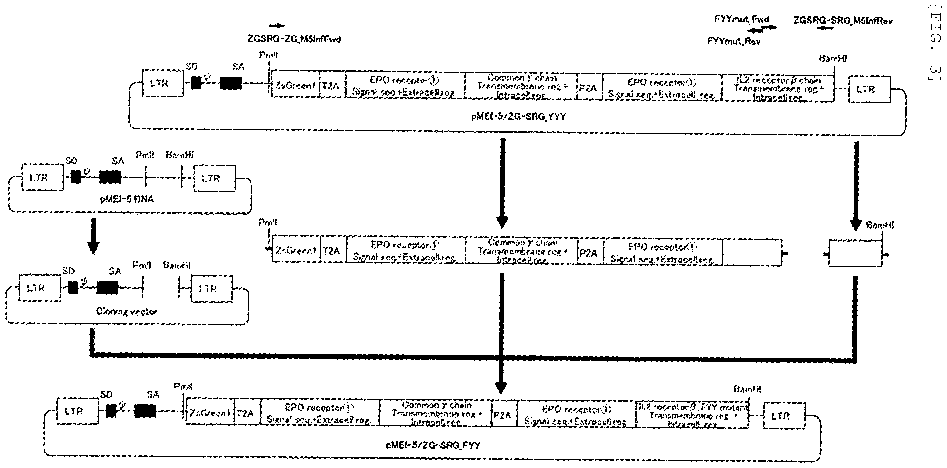

A nucleic acid encoding a fusion polypeptide of an amino acid sequence in which a tyrosine residue at position 1,058 (corresponding to position 381 in the human IL-2 receptor β chain amino acid sequence) was replaced with phenylalanine in the fusion polypeptide (SEQ ID NO: 13) encoded by ZG-SRG_YYY was prepared. This was named ZG-SRG_FYY. The amino acid sequence of this fusion polypeptide is shown in SEQ ID NO: 19. The nucleotide sequence of the nucleic acid is shown in SEQ ID NO: 20. As shown in FIG. 3, the pMEI-5 DNA vector was digested with restriction enzymes PmlI and BamHI to prepare a linearized vector for cloning. Next, PCR was performed using pMEI-5/ZG-SRG_YYY as a template, a ZGSRG-ZG_M5InfFwd primer (SEQ ID NO: 15) and an FYYmut_Rev primer (SEQ ID NO: 21) to obtain an amplified fragment. Similarly, PCR was performed using pMEI-5/ZG-SRG_YYY as a template, an FYYmut_Fwd primer (SEQ ID NO: 22) and a ZGSRG-SRG_M5InfRev primer (SEQ ID NO: 18) to obtain an amplified fragment. These two amplification products were cloned into the linearized vector for cloning as described above using the In-Fusion HD Cloning Kit to prepare retroviral plasmid pMEI-5/ZG-SRG_FYY.

### (6) pMEI-5/ZG-SRG_YFY

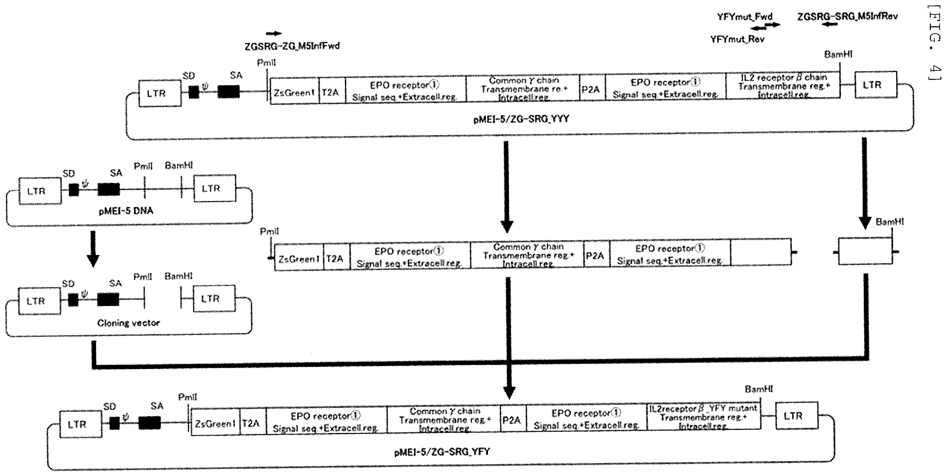

A nucleic acid was prepared encoding a fusion polypeptide of an amino acid sequence in which a tyrosine residue at position 1,061 (corresponding to position 384 in the human IL-2 receptor β chain amino acid sequence) was replaced with phenylalanine in the fusion polypeptide (SEQ ID NO: 13) encoded by ZG-SRG_YYY was prepared. This was named ZG-SRG_YFY. The amino acid sequence of this fusion polypeptide is shown in SEQ ID NO: 23. The nucleotide sequence of the nucleic acid is shown in SEQ ID NO: 24. As shown in FIG. 4, the pMEI-5 DNA vector was digested with restriction enzymes PmlI and BamHI to prepare a linearized vector for cloning. Next, PCR was performed using pMEI-5/ZG-SRG_YYY as a template, a ZGSRG-ZG_M5InfFwd primer (SEQ ID NO: 15) and an YFYmut_Rev primer (SEQ ID NO: 25) to obtain an amplified fragment. Similarly, PCR was performed using pMEI-5/ZG-SRG_YYY as a template, an YFYmut_Fwd primer (SEQ ID NO: 26) and a ZGSRG-SRG_M5InfRev primer (SEQ ID NO: 18) to obtain an amplified fragment. These two amplification products were cloned into the linearized vector for cloning as described above using the In-Fusion HD Cloning Kit to prepare retroviral plasmid pMEI-5/ZG-SRG_YFY.

### (7) pMEI-5/ZG-SRG_YYF

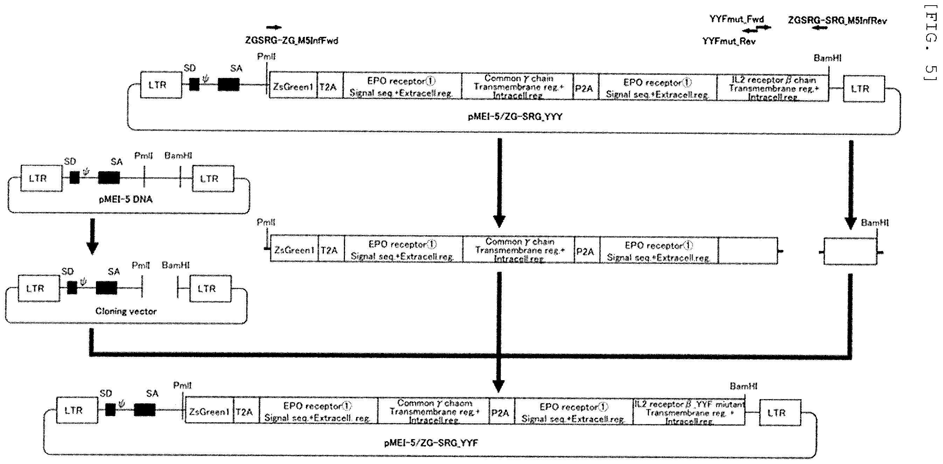

A nucleic acid encoding a fusion polypeptide of an amino acid sequence in which a tyrosine residue at position 1,064 (corresponding to position 387 in the human IL-2 receptor β chain amino acid sequence) was replaced with phenylalanine in the fusion polypeptide (SEQ ID NO: 13) encoded by ZG-SRG_YYY was prepared. This was named ZG-SRG_YYF. The amino acid sequence of this fusion polypeptide is shown in SEQ ID NO: 27. The nucleotide sequence of the nucleic acid is shown in SEQ ID NO: 28. As shown in FIG. 5, the pMEI-5 DNA vector was digested with restriction enzymes PmlI and BamHI to prepare a linearized vector for cloning. Next, PCR was performed using pMEI-5/ZG-SRG_YYY as a template, a ZGSRG-2G M5InfFwd primer (SEQ ID NO: 15) and an YYFmut_Rev primer (SEQ ID NO: 29) to obtain an amplified fragment. Similarly, PCR was performed using pMEI-5/ZG-SRG_YYY as a template, an YYFmut_Fwd primer (SEQ ID NO: 30) and a ZGSRG-SRG_M5InfRev primer (SEQ ID NO: 18) to obtain an amplified fragment. These two amplification products were cloned into the linearized vector for cloning as described above using the In-Fusion HD Cloning Kit to prepare retroviral plasmid pMEI-5/ZG-SRG_YYF.

### (8) pMEI-5/ZG-SRG_FFY

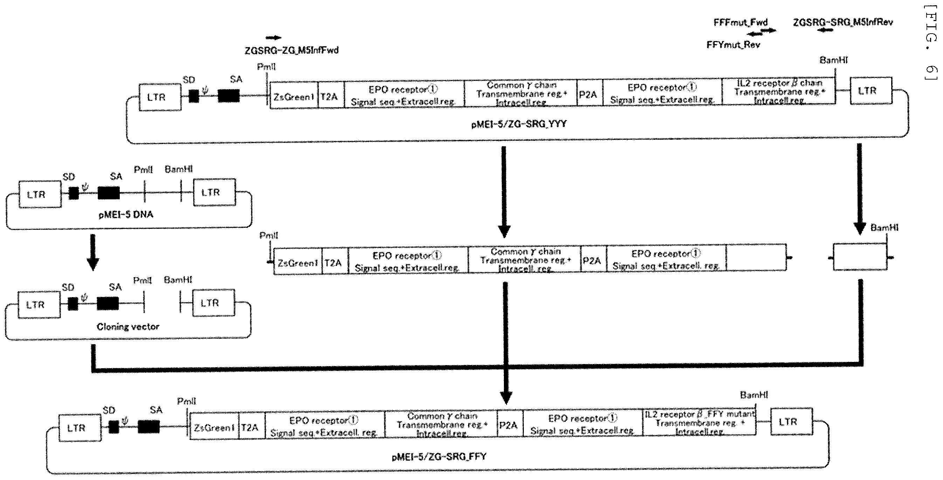

A nucleic acid encoding a fusion polypeptide of an amino acid sequence in which tyrosine residues at positions 1,058 and 1,061 (corresponding to positions 381 and 384 in the human IL-2 receptor β chain amino acid sequence) were replaced with phenylalanine in the fusion polypeptide (SEQ ID NO: 13) encoded by ZG-SRG_YYY was prepared. This was named ZG-SRG_FFY. The amino acid sequence of this fusion polypeptide is shown in SEQ ID NO: 31. The nucleotide sequence of the nucleic acid is shown in SEQ ID NO: 32. As shown in FIG. 6, the pMEI-5 DNA vector was digested with restriction enzymes PmlI and BamHI to prepare a linearized vector for cloning. Next, PCR was performed using pMEI-5/ZG-SRG_FYY as a template, a ZGSRG-ZG_MSInfFwd primer (SEQ ID NO: 15) and an FFYmut_Rev primer (SEQ ID NO: 33) to obtain an amplified fragment. Similarly, PCR was performed using pMEI-S/ZG-SRG_FYY as a template, an FFYmut_Fwd primer (SEQ ID NO: 34) and a ZGSRG-SRG_MSInfRev primer (SEQ ID NO: 18) to obtain an amplified fragment. These two amplification products were cloned into the linearized vector for cloning as described above using the In-Fusion HD Cloning Kit to prepare retroviral plasmid pMEI-5/ZG-SRG_FFY.

### (9) pMEI-5/ZG-SRG_YFF

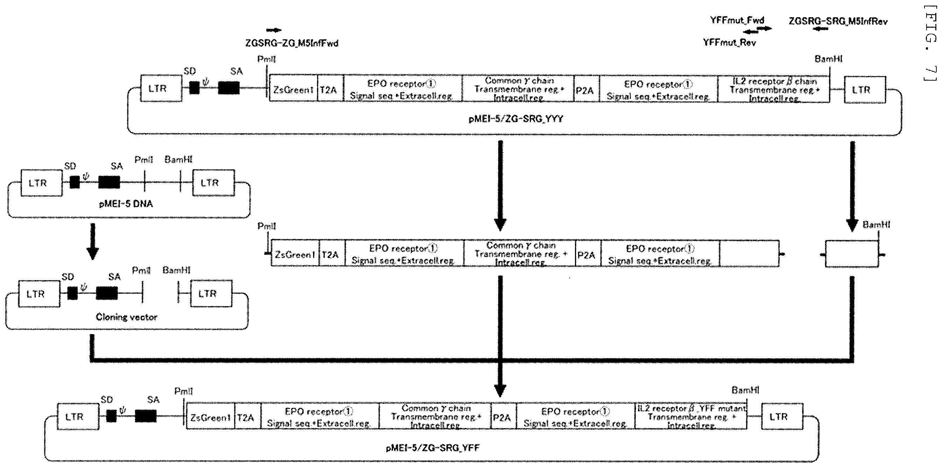

A nucleic acid encoding a fusion polypeptide of an amino acid sequence in which tyrosine residues at positions 1,061 and 1,064 (corresponding to positions 384 and 387 in the human IL-2 receptor β chain amino acid sequence) were replaced with phenylalanine in the fusion polypeptide (SEQ ID NO: 13) encoded by ZG-SRG_YYY was prepared. This was named ZG-SRG_YFF. The amino acid sequence of this fusion polypeptide is shown in SEQ ID NO: 35. The nucleotide sequence of the nucleic acid is shown in SEQ ID NO: 36. As shown in FIG. 7, the pMEI-5 DNA vector was digested with restriction enzymes PmlI and BamHI to prepare a linearized vector for cloning. Next, PCR was performed using pMEI-5/ZG-SRG_YFY as a template, a ZGSRG-ZG_M5InfFwd primer (SEQ ID NO: 15) and an YFFmut_Rev primer (SEQ ID NO: 37) to obtain an amplified fragment. Similarly, PCR was performed using pMEI-5/ZG-SRG_YFY as a template, an YFFmut_Fwd primer (SEQ ID NO: 38) and a ZGSRG-SRG_M5InfRev primer (SEQ ID NO: 18) to obtain an amplified fragment. These two amplification products were cloned into the linearized vector for cloning as described above using the In-Fusion HD Cloning Kit to prepare retroviral plasmid pMEI-5/ZG-SRG_YFF.

### (10) pMEI-5/ZG-SRG_FYF

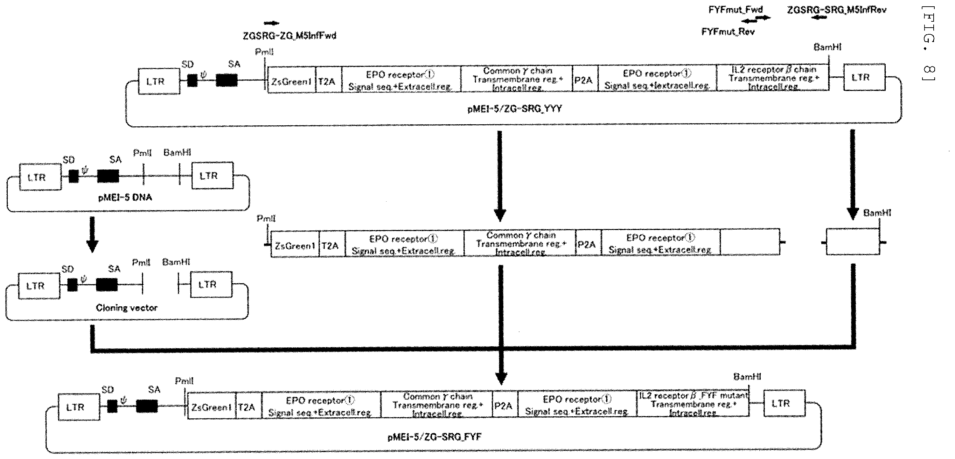

A nucleic acid encoding a fusion polypeptide of an amino acid sequence in which tyrosine residues at positions 1,058 and 1,064 (corresponding to positions 381 and 387 in the human IL-2 receptor β chain amino acid sequence) were replaced with phenylalanine in the fusion polypeptide (SEQ ID NO: 13) encoded by ZG-SRG_YYY was prepared. This was named ZG-SRG_FYF. The amino acid sequence of this fusion polypeptide is shown in SEQ ID NO: 39. The nucleotide sequence of the nucleic acid is shown in SEQ ID NO: 40. As shown in FIG. 8, the pMEI-5 DNA vector was digested with restriction enzymes PmlI and BamHI to prepare a linearized vector for cloning. Next, PCR was performed using pMEI-5/ZG-SRG_FYY as a template, a ZGSRG-ZG_MSInfFwd primer (SEQ ID NO: 15) and an FYFmut_Rev primer (SEQ ID NO: 41) to obtain an amplified fragment. Similarly, PCR was performed using pMEI-S/ZG-SRG_FYY as a template, an FYFmut_Fwd primer (SEQ ID NO: 42) and a ZGSRG-SRG_M5InfRev primer (SEQ ID NO: 18) to obtain an amplified fragment. These two amplification products were cloned into the linearized vector for cloning as described above using the In-Fusion HD Cloning Kit to prepare retroviral plasmid pMEI-5/ZG-SRG_FYF.

### (11) pMEI-5/ZG-SRG_FFF

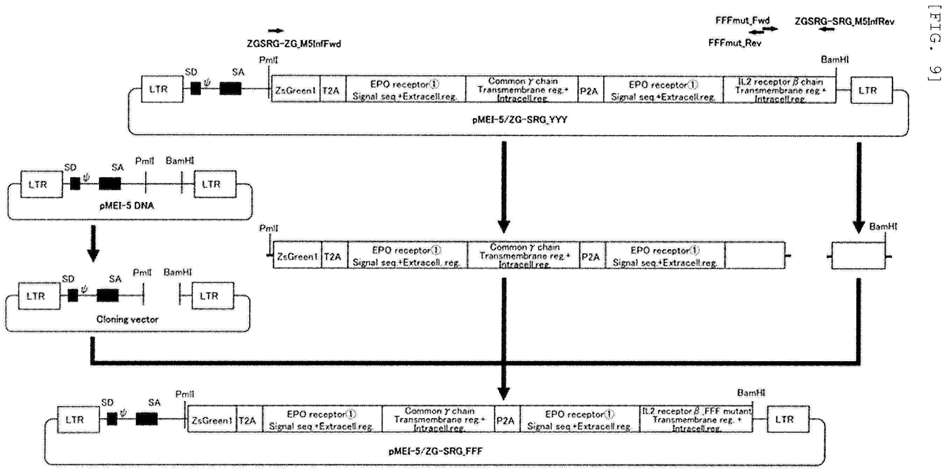

A nucleic acid encoding a fusion polypeptide of an amino acid sequence in which tyrosine residues at positions 1,058, 1,061 and 1,064 (corresponding to positions 381, 384, and 387 in the human IL-2 receptor β chain amino acid sequence) were replaced with phenylalanine in the fusion polypeptide (SEQ ID NO: 13) encoded by ZG-SRG_YYY was prepared. This was named ZG-SRG_FFF. The amino acid sequence of this fusion polypeptide is shown in SEQ ID NO: 43. The nucleotide sequence of the nucleic acid is shown in SEQ ID NO: 44. As shown in FIG. 9, the pMEI-5 DNA vector was digested with restriction enzymes PmlI and BamHI to prepare a linearized vector for cloning. Next, PCR was performed using pMEI-5/ZG-SRG_FYY as a template, a ZGSRG-ZG_M5InfFwd primer (SEQ ID NO: 15) and an FFFmut_Rev primer (SEQ ID NO: 45) to obtain an amplified fragment. Similarly, PCR was performed using pMEI-S/ZG-SRG_FYY as a template, an FFFmut_Fwd primer (SEQ ID NO: 46) and a ZGSRG-SRG_MSInfRev primer (SEQ ID NO: 18) to obtain an amplified fragment. These two amplification products were cloned into the linearized vector for cloning as described above using the In-Fusion HD Cloning Kit to prepare retroviral plasmid pMEI-5/ZG-SRG_FFF.

### (12) pMEI-5/SRG_YYY

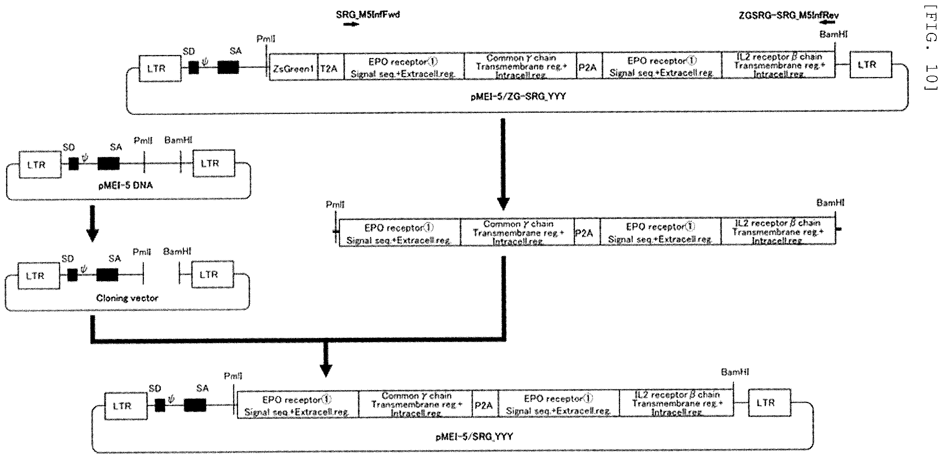

A nucleic acid encoding a fusion polypeptide of an amino acid sequence in which the amino acid sequence of ZsGreen1 and the subsequent T2A sequence were deleted in the fusion polypeptide (SEQ ID NO: 13) encoded by ZG-SRG_YYY was prepared. This was named SRG_YYY. The amino acid sequence of this fusion polypeptide is shown in SEQ ID NO: 47. The nucleotide sequence of the nucleic acid is shown in SEQ ID NO: 48. As shown in FIG. 10, the pMEI-5 DNA vector was digested with restriction enzymes PmlI and BamHI to prepare a linearized vector for cloning. Next, PCR was performed using pMEI-5/ZG-SRG_YYY as a template, an SRG_M5InfFwd primer (SEQ ID NO: 49) and a ZGSRG-SRG_MSInfRev primer (SEQ ID NO: 18) to obtain an amplified fragment. This amplified product was cloned into the linearized vector for cloning as described above using the In-Fusion HD Cloning Kit to prepare retroviral plasmid pMEI-5/SRG_YYY.

### (13) pMEI-5/SRG_FFF

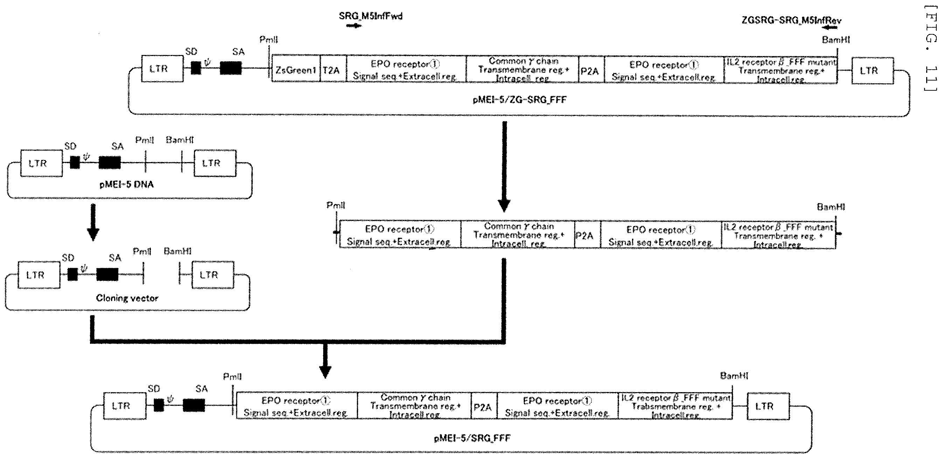

A nucleic acid encoding a fusion polypeptide of an amino acid sequence in which the amino acid sequence of ZsGreen1 and the subsequent T2A sequence were deleted in the fusion polypeptide (SEQ ID NO: 43) encoded by ZG-SRG_FFF was prepared. This was named SRG_FFF. The amino acid sequence of this fusion polypeptide is shown in SEQ ID NO: 50. The nucleotide sequence of the nucleic acid is shown in SEQ ID NO: 51. As shown in FIG. 11, the pMEI-5 DNA vector was digested with restriction enzymes PmlI and BamHI to prepare a linearized vector for cloning. Next, PCR was performed using pMEI-5/ZG-SRG_FFF as a template, an SRG_M5InfFwd primer (SEQ ID NO: 49) and a ZGSRG-SRG_M5InfRev primer (SEQ ID NO: 18) to obtain an amplified fragment. This amplified product was cloned into the linearized vector for cloning as described above using the In-Fusion HD Cloning Kit to prepare retroviral plasmid pMEI-5/SRG_FFF.

### (14) pMEI-5/EL-SRG_FFF

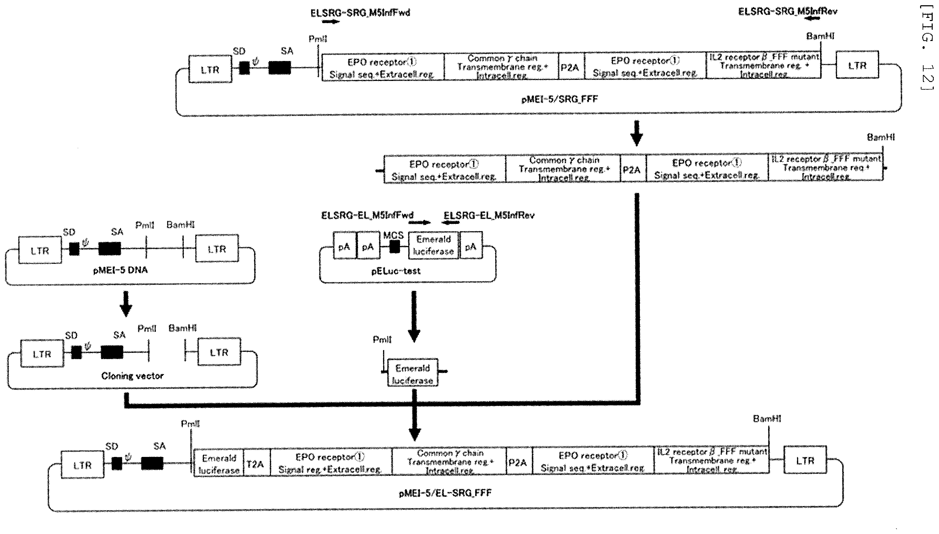

A nucleic acid encoding a seamless fusion polypeptide (Emerald luciferase-T2A-EPOR-IL2Rγ-P2A-EPOR-IL2Rβ) of Emerald luciferase and the fusion polypeptide (SEQ ID NO: 50) encoded by SRG_FFF was prepared. This was named EL-SRG_FFF. The amino acid sequence of this fusion polypeptide is shown in SEQ ID NO: 52. The nucleotide sequence of the nucleic acid is shown in SEQ ID NO: 53. As shown in FIG. 12, the pMEI-5 DNA vector was digested with restriction enzymes PmlI and BamHI to prepare a linearized vector for cloning. Next, PCR was performed using a pELuc-test vector (manufactured by TOYOBO) as a template, an ELSRG-EL_M5InfFwd primer (SEQ ID NO: 54) and an ELSRG-EL_M5InfRev primer (SEQ ID NO: 55) to obtain an amplified fragment. Similarly, PCR was performed using pMEI-5/SRG_FFF as a template, an ELSRG-SRG_MSInfFwd primer (SEQ ID NO: 56) and a ZGSRG-SRG_M5InfRev primer (SEQ ID NO: 18) to obtain an amplified fragment. These two amplification products were cloned into the linearized vector for cloning as described above using the In-Fusion HD Cloning Kit to prepare retroviral plasmid pMEI-5/EL-SRG_FFF.

### (15) pMEI-5/FMC28z

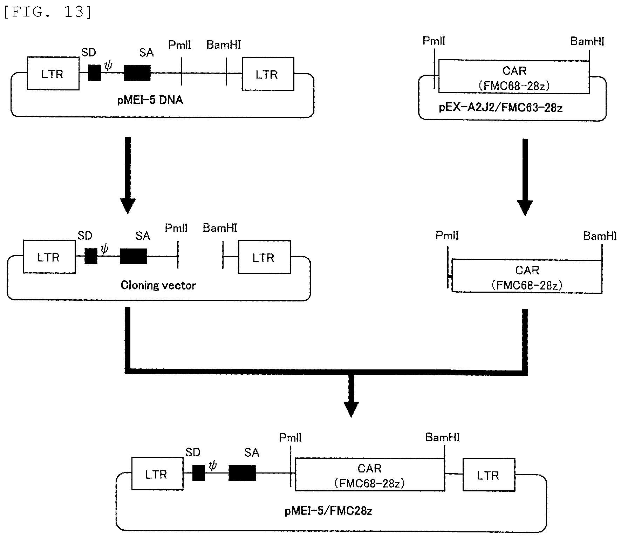

A nucleic acid encoding a polypeptide from position 1 to position 489 (including a signal sequence, an anti-CD19 scFv, a CD28 extracellular domain, a transmembrane domain, an intracellular domain, and a CD3ζ intracellular domain) in the FMC63-28Z chimeric antigen receptor amino acid sequence shown by GenBank: ADM64594.1 was prepared. This was named FMC28z. The amino acid sequence of this fusion polypeptide is shown in SEQ ID NO: 57. The nucleotide sequence of the nucleic acid is shown in SEQ ID NO: 58. This nucleic acid was synthesized as an artificial gene (codon optimized) and cloned into the pEX-A2J2 vector to prepare plasmid pEX-A2J2/FMC28z. Next, as shown in FIG. 13, pEX-A2J2/FMC28z was digested with restriction enzymes PmlI and BamHI to prepare an insert DNA. The insert DNA was then inserted into a linearized pMEI-5 DNA vector obtained by treatment with restriction enzymes PmlI and BamHI to prepare retroviral plasmid pMEI-5/FMC28z.

### (16) pMEI-5/FMC28z-SRG_FFF

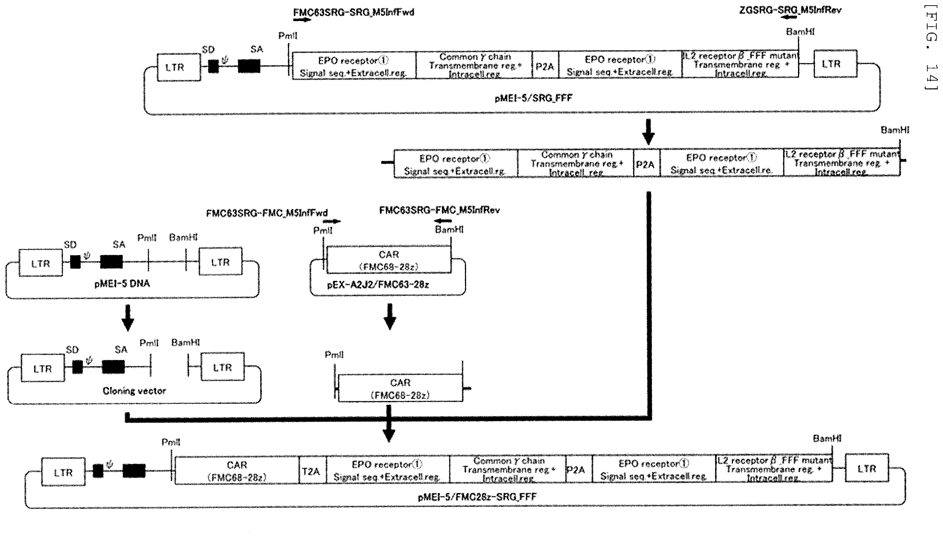

A nucleic acid encoding a seamless fusion of the FMC63-28Z chimeric antigen receptor (SEQ ID NO: 57) and the fusion polypeptide (SEQ ID NO: 50) encoded by SRG_FFF was prepared. This was named FMC28z-SRG_FFF. The amino acid sequence of this fusion polypeptide is shown in SEQ ID NO: 59. The nucleotide sequence of the nucleic acid is shown in SEQ ID NO: 60. As shown in FIG. 14, the pMEI-5 DNA vector was digested with restriction enzymes PmlI and BamHI to prepare a linearized vector for cloning. Next, PCR was performed using pMEI-5/FMC63-28z as a template, an FMC63SRG-FMC_M5InfFwd primer (SEQ ID NO: 61) and an FMC63SRG-FMC_M5InfRev primer (SEQ ID NO: 62) to obtain an amplified fragment. Similarly, PCR was performed using pMEI-5/SRG_FFF as a template, an FMC63SRG-SRG_M5InfFwd primer (SEQ ID NO: 63) and a ZGSRG-SRG_M5InfRev primer (SEQ ID NO: 18) to obtain an amplified fragment. These two amplification products were cloned into the linearized vector for cloning as described above using the In-Fusion HD Cloning Kit to prepare retroviral plasmid pMEI-5/FMC28z-SRG_FFF.

### (17) pEX-A2J2/EpoRm

A nucleic acid encoding a human erythropoietin receptor truncated mutant consisting of a polypeptide from position 2 to position 438 in the human erythropoietin receptor amino acid sequence shown by NCBI Reference Sequence: NP_000112.1 as described in Blood, Vol. 135(9):668-679 (2020) was prepared. This was named EpoRm. The amino acid sequence of this polypeptide is shown in SEQ ID NO: 64. The nucleotide sequence of the nucleic acid is shown in SEQ ID NO: 65. This nucleic acid was synthesized as an artificial gene (codon optimized) and cloned into the pEX-A2J2 vector to prepare plasmid pEX-A2J2/EpoRm.

### (18) pMEI-5/ZG-EpoRm

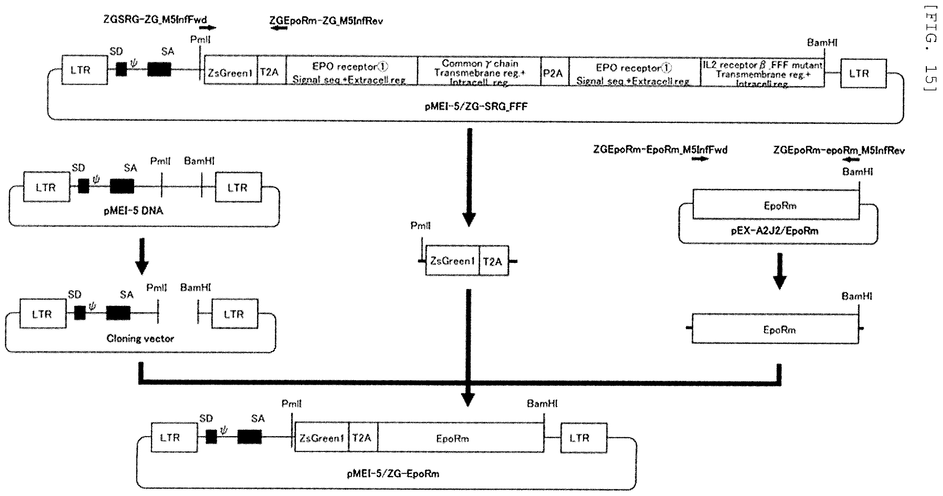

A nucleic acid encoding a seamless fusion polypeptide of ZsGreen1 and the truncated mutant of the human erythropoietin receptor (SEQ ID NO: 64) was prepared. This was named ZG-EpoRm. The amino acid sequence of this polypeptide is shown in SEQ ID NO: 66. The nucleotide sequence of the nucleic acid is shown in SEQ ID NO: 67. As shown in FIG. 15, the pMEI-5 DNA vector was digested with restriction enzymes PmlI and BamHI to prepare a linearized vector for cloning. Next, PCR was performed using pMEI-5/ZG-SRG_FFF as a template, a ZGSRG-ZG_M5InfFwd primer (SEQ ID NO: 15) and a ZGEpoRm-ZG_M5InfRev primer (SEQ ID NO: 68) to obtain an amplified fragment. Similarly, PCR was performed using pEX-A2J2/EpoRm as a template, a ZGEpoRm-EpoRm_M5InfFwd primer (SEQ ID NO: 69), and a ZGEpoRm-EpoRm_MSInfRev primer (SEQ ID NO: 70) to obtain an amplified fragment. These two amplification products were cloned into the linearized vector for cloning as described above using the In-Fusion HD Cloning Kit to prepare retroviral plasmid pMEI-5/ZG-EpoRm.

### Example 2. Preparation of retroviral vector solution

Unless otherwise specified, a 293T cell line and a PG13 cell line were cultured in a DMEM/F-12 medium supplemented with 10% fetal bovine serum (FBS) and 100 U/mL penicillin-streptomycin.

Retroviral vectors corresponding to the various retroviral plasmids described in Example 1 were prepared by procedures as described below. First, transient retroviral vector supernatants were prepared using 293T cells. 293T cells were transfected with retroviral packaging plasmids [Gag-pol expression plasmid pGP, VSV-G expression plasmid pVSV-G (Uchibori R, et al. Mol Ther Oncolytics. 2018;12:16-25.)] and the retroviral plasmid (pMEI-5/ZG-SRG_YYY, pMEI-5/ZG-SRG_FYY, pMEI-5/ZG-SRG_YFY, pMEI-5/ZG-SRG_YYF, pMEI-5/ZG-SRG_FFY, pMEI-5/ZG-SRG_YFF, pMEI-5/ZG-SRG_FYF, pMEI-5/ZG-SRG_FFF, pMEI-5/SRG_YYY, pMEI-5/SRG_FFF, pMEI-5/EL-SRG_FFF, pMEI-5/FMC28z, pMEI-5/FMC28z-SRG_FFF, or pMEI-5/ZG-EpoRm) by the calcium phosphate method, and cultured at 37°C in 5% CO₂ for 7 hours. The entire medium was replaced with DMEM/F-12 supplemented with a final concentration 5 mM of sodium butyrate (STEMCELL Technologies Inc.), and the cells were cultured for 16 hours in a 37°C, 5% CO₂ incubator. The entire medium was replaced with DMEM/F-12, and the cells were cultured at 32°C in 5% CO₂ for 24 hours. Then, the cells were centrifuged at 2,000×g for 10 minutes to collect the viral vector supernatant, which was then filtered through a 0.45 µm filter to remove cell debris. The viral supernatant was further purified using a Retrovirus Purification Mini Kit (ViraTrap, Biomiga, Inc.).

Next, retroviral vectors were produced using the PG13 cell line as a packaging cell line. Specifically, PG13 cells seeded in a 6-well plate were exposed to the purified virus solution in the presence of 8 µg/mL polybrene, and left to stand overnight in an incubator set at 37°C and 5% CO₂. The next day, the entire medium was replaced with fresh medium, and the cells were left to stand for 6 hours in a 37°C, 5% CO₂ incubator. The transduced cells were detached, and all were subcultured in a T-75 flask. To isolate single-cell clones by limiting dilution when the cells reached subconfluence, the cells were cultured for 7 to 10 days in a 37°C, 5% CO₂ incubator. The cells were detached from the wells by trypsinization, seeded onto a 12-well plate, and cultured for 3 to 4 days in a 37°C, 5% CO₂ incubator. The cells were detached from the wells by trypsinization, and 3/4 of the cells were resuspended in freezing medium (CELLBANKER; Nippon Zenyaku Kogyo Co., Ltd.), frozen, and stored in a -80°C freezer. The remaining 1/4 of the cells were seeded onto a 12-well plate and cultured for 3 to 4 days in a 37°C, 5% CO₂ incubator. When the cells reached confluence, the medium was completely replaced with DMEM/F-12 supplemented with sodium butyrate at a final concentration of 5 mM. The plate was left to stand for 48 hours in a 37°C, 5% CO₂ incubator. After centrifugation at 2,000×g for 10 minutes, the supernatant was collected and filtered through a 0.45 µm filter to remove cell debris. Thus, a retroviral vector solution was obtained. A portion of the retrovirus vector solution was used to measure the titer [Retrovirus Titer Set (for Real Time PCR), manufactured by TAKARA BIO INC.], and the remainder was dispensed into cryo-ceramic tubes, frozen, and stored in a -80°C freezer until use. The viral supernatant titer of each clone [Retrovirus Titer Set (for Real Time PCR)] was measured and selected to establish a clone line for stably producing the retroviral vector. For retroviral vector stable producing clone lines with a viral titer of 4.0 x 10⁹ copies/mL or more in the supernatant, the cryopreserved cells were thawed and seeded into T-75 flasks and then expanded to T-225 flasks. When the cells reached confluence, the medium was replaced with DMEM/F-12 supplemented with 5 mM (final concentration) sodium butyrate. A retroviral vector solution was collected using the same procedure as described above, dispensed into cryo-ceramic tubes, frozen, and stored in a -80°C freezer until use.

### Example 3. Comparison of proliferation efficiency of SRG construct-introduced cells

Hereinafter, a nucleic acid (including a retrovirus harboring the nucleic acid) encoding the T2A-EPOR-IL2Rγ-P2A-EPOR-IL2Rβ fusion polypeptide is referred to as prototype SRG, and a nucleic acid encoding the fusion polypeptide with a mutation in the intracellular region of the IL-2 receptor β chain (FYY, YFY, YYF, FFY, YFF, FYF or FFF) is referred to as mutant SRG.

### (1) Transduction of SRG constructs into T cells

Peripheral blood was collected from a volunteer who provided informed consent into a BD Vacutainer CPT mononuclear cell collection tube, and centrifuged at 25°C and 1,500 x g for 15 minutes to separate peripheral blood mononuclear cells (PBMCs) and plasma. PBMCs were washed twice with CELLOTION (Nippon Zenyaku Kogyo Co., Ltd.), mixed with a mixture of cell cryopreservation medium CP-1 (Kyokuto Pharmaceutical Industrial Co., Ltd.), human serum albumin (HSA) (Albuminar; CSL Behring), and RPMI, and cryopreserved at -80°C until use. The plasma was heat-inactivated in a water bath set at 56°C for 30 minutes, and then cryopreserved at -80°C until use.

To activate the PBMCs, 5 µg/mL CD3 Monoclonal Antibody (OKT3) (Thermo Fisher Scientific) diluted with ACD-A solution (Terumo) and 20 µg/mL RetroNectin (TAKARA BIO INC.) were added to a surface-untreated 6-well plate at 1 mL/well, and the plate was left for 3 hours in a 37°C, 5% CO₂ incubator, and at 4°C overnight to prepare an OKT3/RetroNectin-coated plate. The PBMCs were thawed, suspended at 2.0 × 10⁵ cells/mL in a medium [a GT-T551 medium (TAKARA BIO INC.) supplemented with 175 IU/mL IL-2 and 0.1% heat-inactivated autologous plasma], and washed thrice with DMEM/F-12 medium (not containing FBS and penicillin-streptomycin). Into the OKT3/RetroNectin-coated plate, 6.5 mL/well of the cell suspension was added. The plate was left for 4 days in a 37°C, 5% CO₂ incubator to promote the activation and proliferation of T cells in PBMCs.

For transduction into T cells, a RetroNectin-conjugated viral transduction method [J Biochem. 2001 Sep;130(3): 331-4] was used. Into a surface-untreated 6-well plate, 2 mL/well of a 20 µg/mL solution of RetroNectin diluted with PBS was added, and the plate was left at 4°C. The RetroNectin solution was removed, and 2 mL/well of a blocking solution [phosphate-buffered saline (PBS) containing 2% bovine serum albumin (BSA)] was added. The plate was left to stand at room temperature for 30 minutes for blocking treatment. The blocking solution was removed, and 2 mL/well of PBS was added to wash the plate once. Then, the PBS was removed. The retroviral vector solution (diluted to 4.0 × 10⁹ copies/mL with PBS) carrying prototype SRG or mutant SRG prepared in Example 2 was added at 4 mL/well onto the RetroNectin-coated plate. The plate containing the retrovirus was centrifuged at 1,960 x g for 2 hours at 32°C to promote adsorption of the virus particles onto RetroNectin. The viral solution was removed from the plate and washed with 2 mL of PBS supplemented with 1% BSA. OKT3/RetroNectin-activated T cells (diluted to 4.0 × 10⁵ cells/mL in GT-T551 medium supplemented with 175 IU/mL IL-2 and 0.1% heat-inactivated autologous plasma) were added to the virus-adsorbed plate at 4 mL/well, and centrifuged at 1,960 x g for 10 minutes at 32°C. The plate was left overnight in a 37°C, 5% CO₂ incubator to carry out the first transduction. The transduced T cells were collected the next day and washed with the above-mentioned medium. After the supernatant was removed, the cells were resuspended in 4 mL of a medium and added to a virus-adsorbed plate prepared in the same way as described above. The plate was left overnight in an incubator set at 37°C and 5% CO₂ to perform the second transduction.

### (2) Comparison of proliferation efficiency of SRG construct-transduced cells

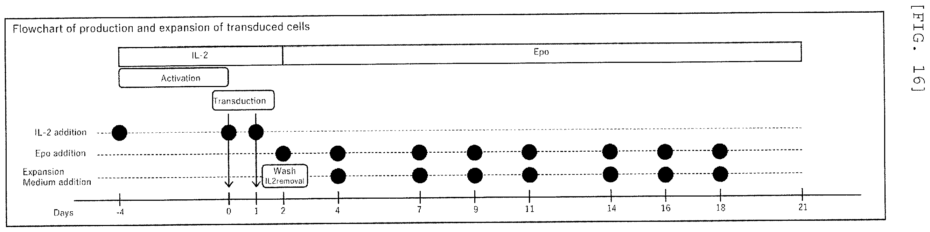

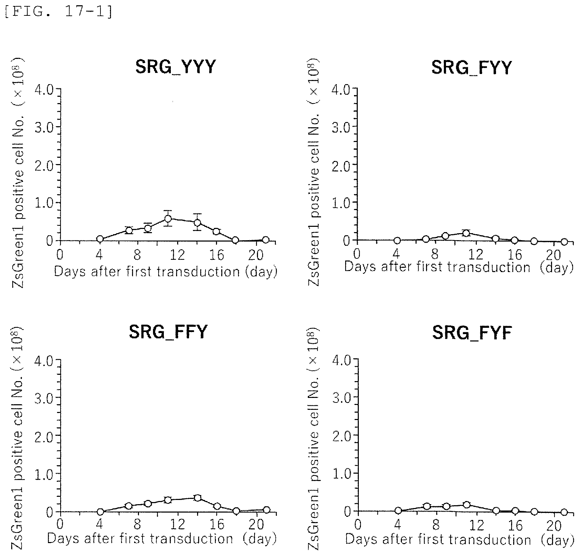

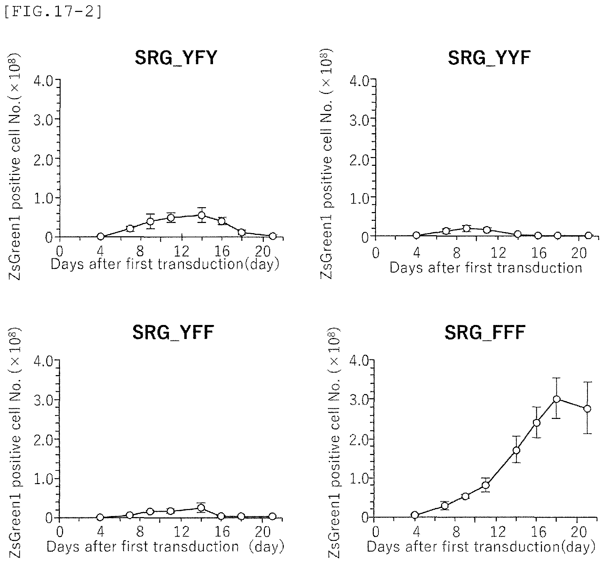

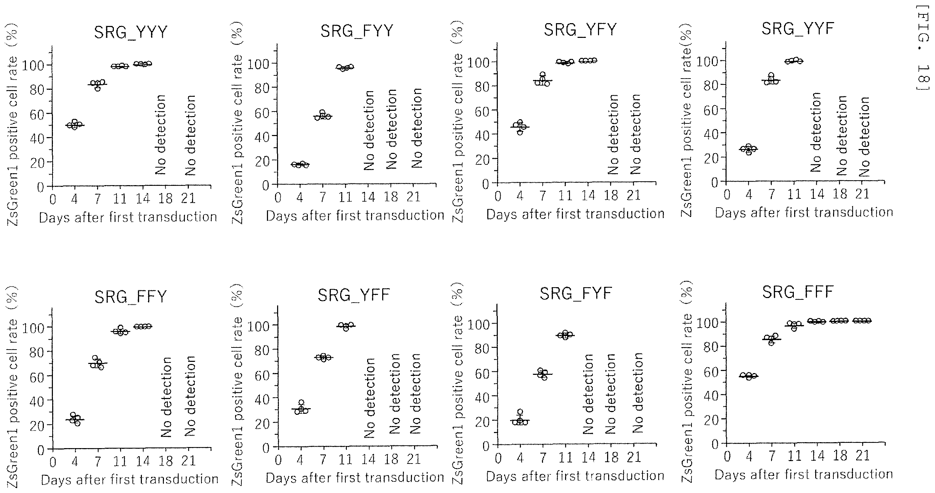

As shown in FIG. 16, the transduced T cells were collected the next day and washed twice with a basal medium (a GT-T551 medium supplemented with 0.1% heat-inactivated autologous plasma). The transduced cells were resuspended in 20 mL of the basal medium supplemented with 9.0 IU/mL erythropoietin (EPO), and left to stand in an incubator set at 37°C and 5% CO₂ (Day 2). On days 4, 7, 9, 11, 14, 16, and 18, an equal volume of basal medium and 9.0 IU/mL EPO were added. FIG. 17-1 and FIG. 17-2 show growth curves of cells (ZsGreen1-positive cells) transfected with prototype SRG and various mutant SRG. FFF-mutant SRG-transduced cells showed EPO-dependent proliferation over a period of 3 weeks after transduction. FIG. 18 shows ZsGreen1 positive rates. In all transduced cell groups, the addition of EPO induced selective proliferation of the transduced cells, and at the end point, a cell population consisting almost entirely of the transduced cells was obtained. FFF-mutant SRG-transduced cells showed proliferation of ZsGreen1-positive cells over a period of 3 weeks after transduction and EPO-concentration-dependent proliferation.

### Example 4. Optimization of amount of EPO added

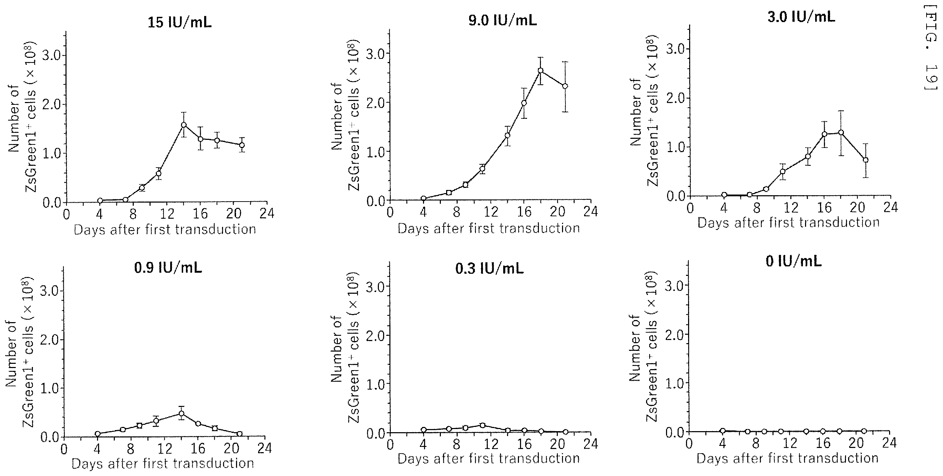

T cells stimulated with OKT3/RetroNectin were transduced with FFF mutant SRG by the RetroNectin-conjugated virus infection method. The T cells that completed the second transduction were collected the next day (Day 2) and washed twice with basal medium. The transduced cells were resuspended in 20 mL of the basal medium supplemented with 0, 0.3, 0.9, 3.0, 9.0, or 15 IU/mL EPO, and left to stand in an incubator set at 37°C and 5% CO₂ (Day 2). On days 4, 7, 9, 11, 14, 16 and 18, an equal volume of the basal medium was added, and 0, 0.3, 0.9, 3.0, 9.0, or 15 IU/mL EPO was added. FIG. 19 shows the growth curves of transduced cells (ZsGreen1-positive cells). After sufficient stirring, 100 µL of the culture medium was collected, washed twice with CELLOTION, and resuspended in 100 µL PBS. The cell suspension was mixed with 5 µL of a staining solution prepared by diluting 1 mM Ethidium Homodimer III solution 50-fold with PBS, and incubated at room temperature for 15 minutes under light shielding to stain dead cells. Then, 10 µL of the reaction solution was transferred to Countess Cell Counting Chamber Slides (Thermo Fisher Scientific), and the numbers of ZsGreen1-positive and dead cells were counted using Countess II FL (Thermo Fisher Scientific). ZsGreen1-positive cells proliferated in an EPO-concentration-dependent manner over a period of 3 weeks after transduction.

### Example 5. Comparison of proliferation of transduced cells depending on amount of gene transfer vector

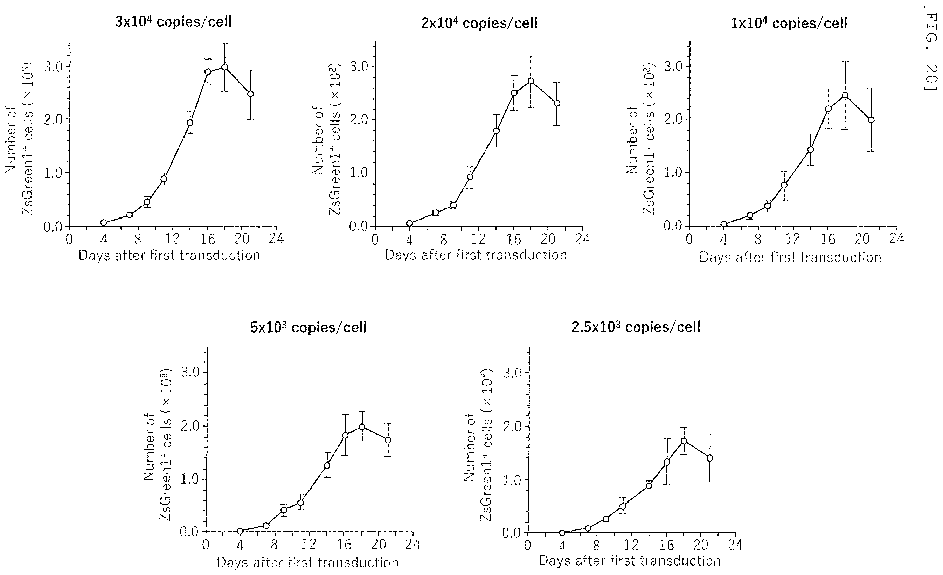

T cells stimulated with OKT3/RetroNectin were transduced with FFF mutant SRG by the RetroNectin-conjugated virus infection method. The virus concentrations at the time of transduction were 2.5 x 10³, 5.0 x 10³, 1.0 x 10⁴, 2.0 x 10⁴, and 3.0 x 10⁴ copies/cell. The T cells that completed the second transduction were collected the next day (Day 2) and washed twice with the basal medium. The transduced cells were resuspended in 20 mL of basal medium supplemented with 9.0 IU/mL EPO and left to stand in an incubator set at 37°C and 5% CO₂ (Day 2). On days 4, 7, 9, 11, 14, 16, and 18, an equal volume of basal medium, and 9.0 IU/mL EPO were added. FIG. 20 shows the growth curves of transduced cells (ZsGreen1-positive cells). After sufficient stirring, 100 µL of the culture medium was collected, washed twice with CELLOTION, and resuspended in 100 µL PBS. The cell suspension was mixed with a staining solution (5 µL) prepared by diluting 1 mM Ethidium Homodimer III solution 50-fold with PBS, and incubated at room temperature for 15 minutes under light shielding to stain dead cells. Then, 10 µL of the reaction mixture was transferred to Countess Cell Counting Chamber Slides, and the numbers of ZsGreen1-positive and dead cells were counted using Countess II FL. The proliferation of ZsGreen1 positive cells over a period of 3 weeks after transduction correlated with the amount of vector used for transduction.

### Example 6. Comparison of proliferation with prior art

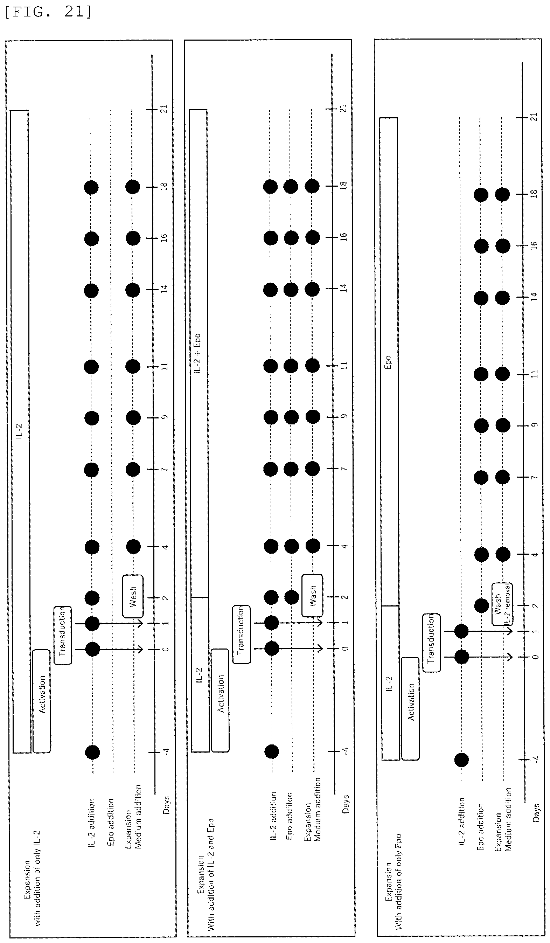

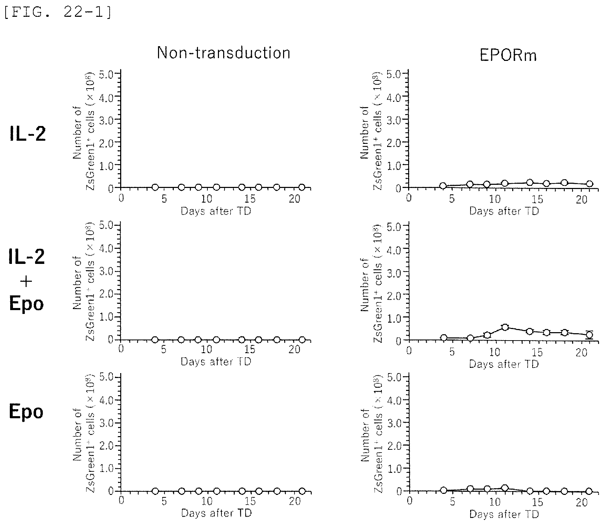

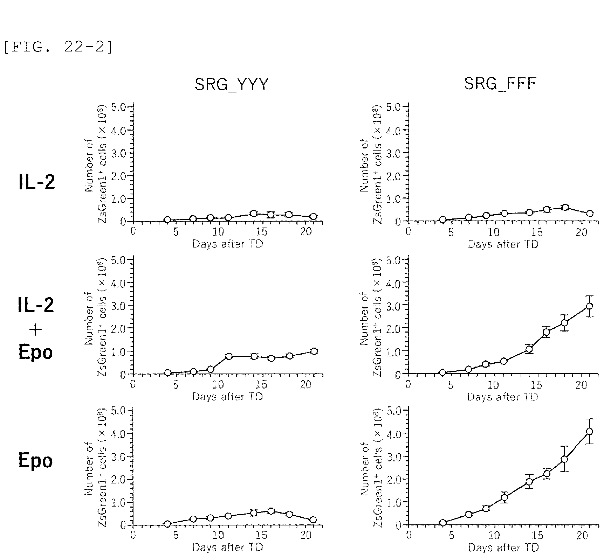

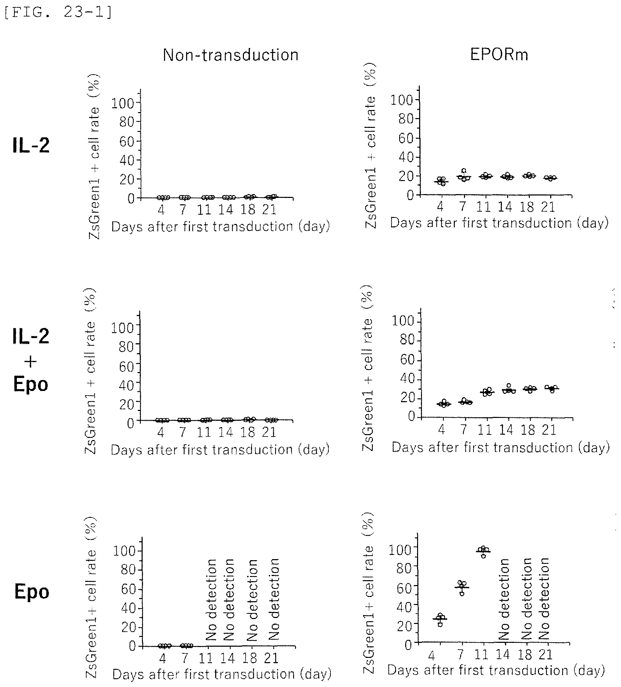

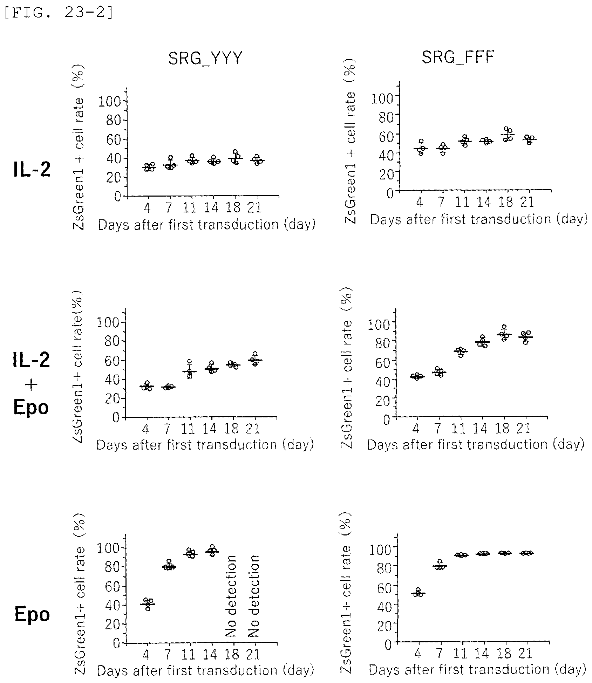

T cells stimulated with OKT3/RetroNectin were transduced with the YYY prototype SRG, FFF mutant type SRG, or EpoRm by the RetroNectin-conjugated virus infection method. The T cells that completed the second transduction were collected the next day (Day 2) and washed twice with basal medium. As shown in FIG. 21, in the culture system containing only IL-2, the transduced cells were resuspended in 30 mL of basal medium supplemented with IL-2 at a final concentration of 175 IU/mL, and left to stand in an incubator set at 37°C and 5% CO₂ (Day 2). On days 4, 7, 9, 11, 14, 16, and 18, an equal volume of basal medium supplemented with IL-2 at a final concentration of 175 IU/mL were added. In the culture system containing combination of IL-2 and EPO, the transduced cells were resuspended in 30 mL of basal medium supplemented with IL-2 at a final concentration of 175 IU/mL and EPO at a final concentration of 9.0 IU/mL, and left to stand in an incubator set at 37°C and 5% CO₂ (Day 2). On days 4, 7, 9, 11, 14, 16, and 18, an equal volume of basal medium supplemented with IL-2 at a final concentration of 175 IU/mL and EPO at a final concentration of 9.0 IU/mL was added. In the culture system containing only EPO, the transduced cells were resuspended in 20 mL of basal medium supplemented with EPO at a final concentration of 9.0 IU/mL, and left to stand in an incubator set at 37°C and 5% CO₂ (Day 2). On days 4, 7, 9, 11, 14, 16, and 18, an equal volume of basal medium supplemented with EPO at a final concentration of 9.0 IU/mL was added. In addition, as a control, non-transduced T cells were cultured in each of the aforementioned culture systems. FIG. 22-1 and FIG. 22-2 show the growth curves of the transduced cells (ZsGreen1-positive cells). After sufficient stirring, 100 µL of the culture medium was collected, washed twice with CELLOTION, and resuspended in 100 µL PBS. The cell suspension was mixed with 5 µL of a staining solution prepared by diluting 1 mM Ethidium Homodimer III solution 50-fold with PBS, and incubated at room temperature for 15 minutes under light shielding to stain dead cells. Then, 10 µL of the reaction mixture was transferred to Countess Cell Counting Chamber Slides, and the numbers of ZsGreen1-positive and dead cells were counted using Countess II FL. ZsGreen1-positive cells proliferated in an EPO-concentration-dependent manner over a period of 3 weeks after transduction. The measurement results of ZsGreen1 positive rates using a flow cytometer are shown in FIG. 23-1 and FIG. 23-2. In all transduced cell groups, the addition of EPO induced selective proliferation of the transduced cells, and at the end point, a cell population consisting almost entirely of the transduced cells was obtained. FFF-mutant SRG-transduced cells showed proliferation and survival of ZsGreen1 positive cells over a period of 3 weeks after transduction and EPO-concentration-dependent proliferation.

### Example 7. EPO-dependent in vivo proliferation of transduced cells infused into immunodeficient mouse

### (1) Comparison of in vivo proliferation of SRG construct-transduced cells at different doses of EPO

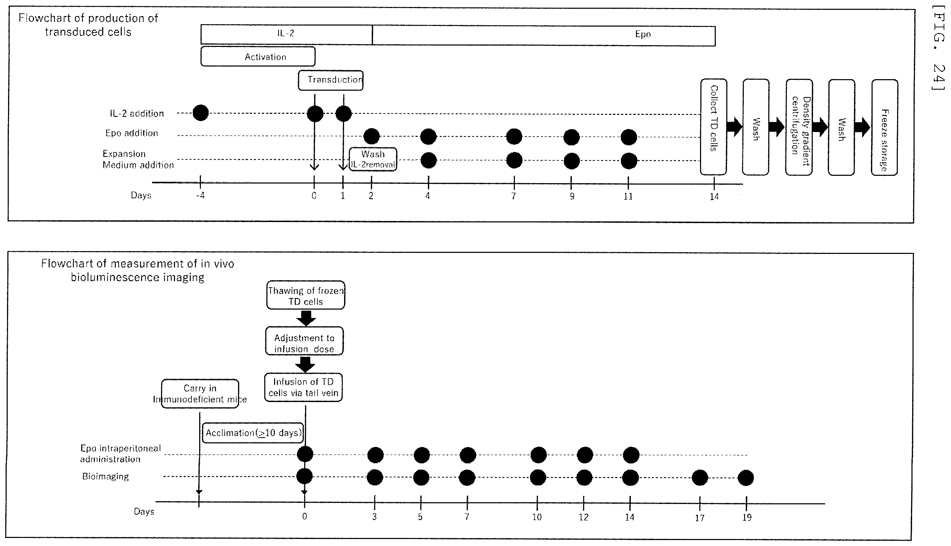

As shown in FIG. 24, T cells stimulated with OKT3/RetroNectin were transduced with Emerald luciferase-loaded FFF mutant SRG (a retroviral vector prepared using pMEI-5/EL-SRG_FFF) by the RetroNectin-conjugated virus infection method. The T cells that had completed the second transduction were collected the next day (Day 2) and washed twice with the basal medium. The transduced cells were resuspended in 10 mL of the basal medium supplemented with 9.0 IU/mL EPO, and left to stand in an incubator set at 37°C and 5% CO₂ (Day 2). On days 4, 7, 9 and 11, an equal volume of the basal medium was added, and 9.0 IU/mL EPO was added. On day 14, the cells were harvested and washed with CELLOTION. Then, dead cells were removed by density gradient centrifugation using a human lymphocyte separating solution (Lympholyte-H; Cedarlane Laboratories Ltd.). A target cell layer was collected and washed twice with CELLOTION. Then, the cells were mixed with a mixture of cell cryopreservation medium CP-1, HSA (Albuminar) and RPMI, and cryopreserved at -80°C until use.

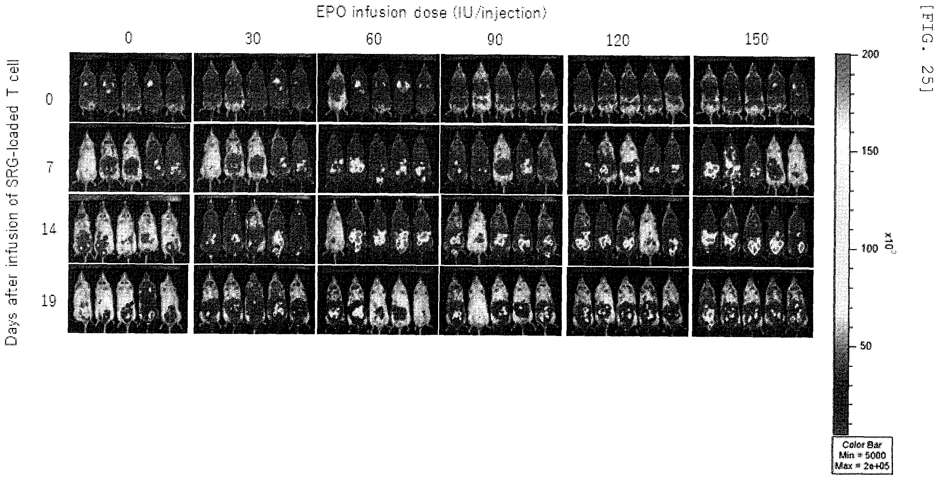

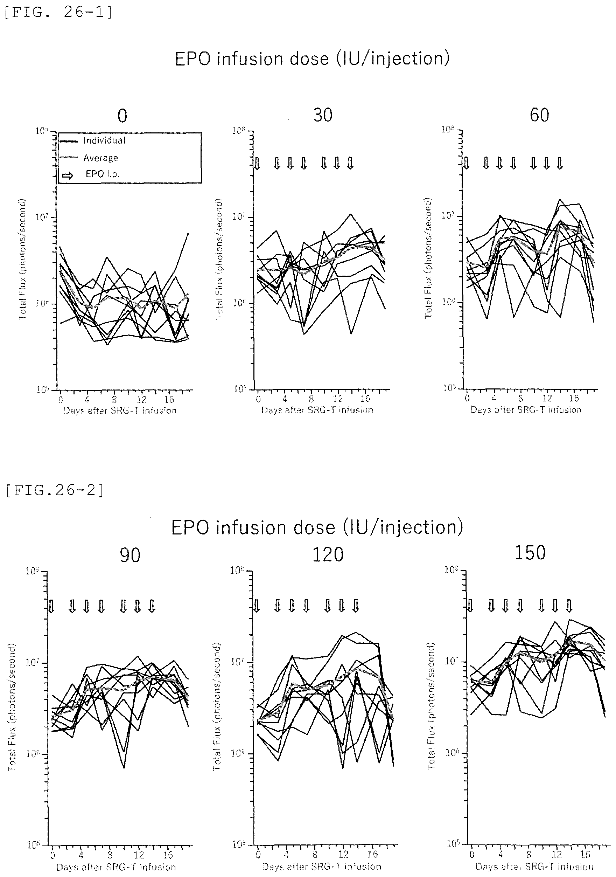

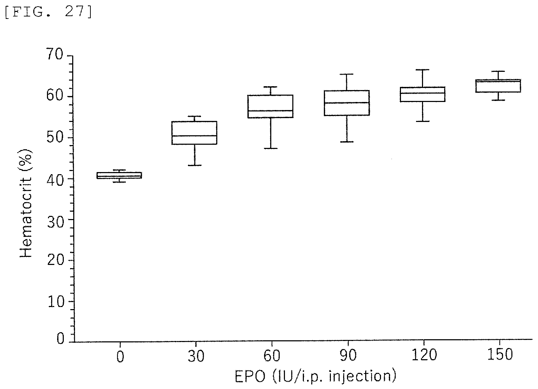

Immunodeficient NOG tumor-bearing mice were infused with 1 × 10⁶ SRG-FFF-loaded T cells via the tail vein (Day 0). On days 0, 3, 5, 7, 10, 11, and 14, 0, 30, 60, 90, 120, and 150 IU/injection of EPO were administered intraperitoneally. *In vivo* bioluminescence imaging of luciferase activity over time after transfusion of the transduced T cells is shown in FIG. 25, and graphs of quantified luminescence intensity are shown in FIG. 26-1 and FIG. 26-2. It was shown that the infused SRG-loaded T cells were proliferate in an EPO dose-dependent manner. As shown in FIG. 27, the hematocrit values of the whole blood collected on Day 17 showed an EPO dose-dependent increase.

### (2) Comparison of in vivo proliferation of SRG construct-introduced cells depending on EPO administration period

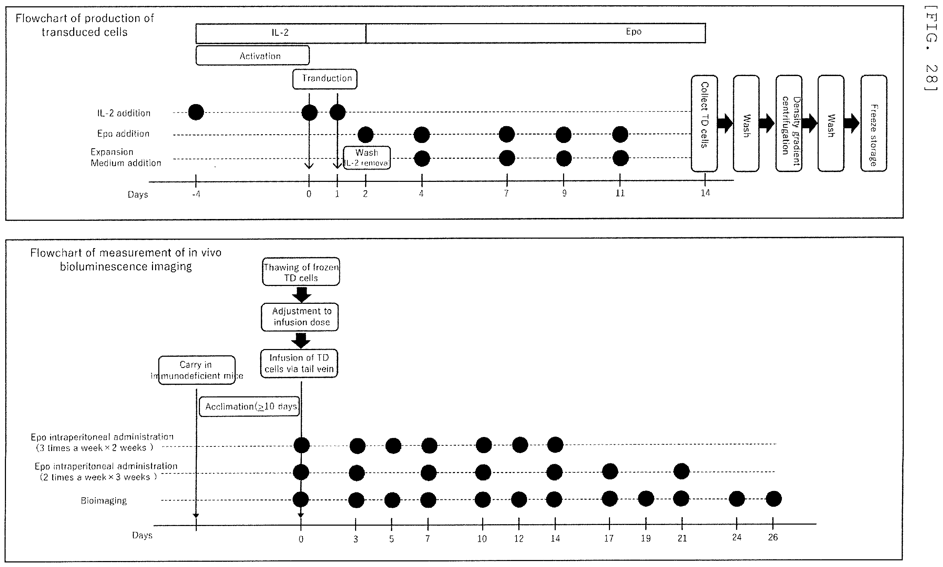

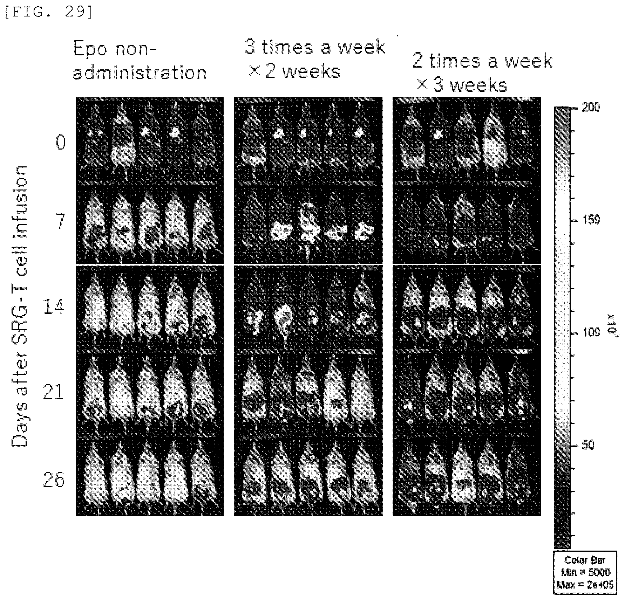

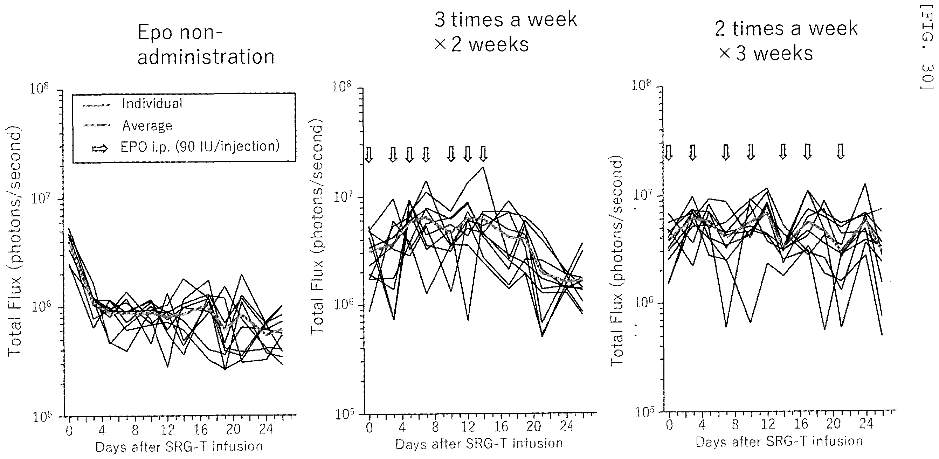

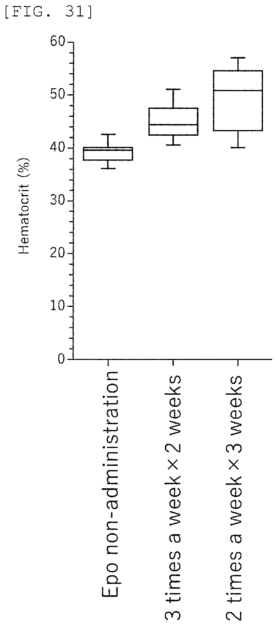

As shown in FIG. 28, immunodeficient NOG tumor-bearing mice were infused with 1 × 10⁶ SRG-FFF-loaded T cells via the tail vein (Day 0). On days 0, 3, 5, 7, 10, 12, and 14 (three times a week for two weeks) or on days 0, 3, 7, 10, 14, 17, and 21 (twice a week for three weeks), 90 IU/injection of EPO was administered intraperitoneally. In *vivo* bioluminescence imaging of luciferase activity over time after transfusion of the transduced T cells is shown in FIG. 29, and graphs of the quantified luminescence intensity are shown in FIG. 30. The *in vivo* proliferation of infused SRG-loaded T cells was EPO dose-dependent under all conditions. The "twice a week for three weeks" group showed sustained proliferation and survival of the infused cells. As shown in FIG. 31, the hematocrit values of the whole blood collected on Day 26 showed an EPO-dependent increase.

### Example 8. Measurement of immunophenotype of transduced T cells

### (1) Transduction of SRG-loaded CAR construct into T cells

T cells stimulated with OKT3/RetroNectin were transduced with CAR (a retroviral vector prepared using pMEI-5/FMC28z) or SRG-FFF-loaded CAR (a retroviral vector prepared using pMEI-5/FMC28z-SRG_FFF) by the RetroNectin-conjugated virus infection method. The T cells that had completed the second transduction were collected the next day (Day 2) and washed twice with the basal medium. The non-transduced cells and the CAR-transduced cells were suspended in 30 mL of the basal medium supplemented with IL-2 at a final concentration of 175 IU/mL, and left to stand in an incubator set at 37°C and 5% CO₂ (Day 2). On days 4, 7, 9, 11, 14, 16, and 18, an equal volume of the basal medium supplemented with IL-2 at a final concentration of 175 IU/mL was added. The cells transduced with the SRG-loaded CAR were resuspended in 10 mL of the basic medium supplemented with EPO at a final concentration of 9.0 IU/mL, and left to stand in an incubator set at 37°C and 5% CO₂ (Day 2). On days 4, 7, 9 and 11, an equal volume of the basal medium supplemented with 9.0 IU/mL EPO was added. On day 14, the cells were harvested and washed with CELLOTION. Then, dead cells were removed by density gradient centrifugation using a human lymphocyte separating solution (Lympholyte-H). A target cell layer was collected, and washed twice with CELLOTION. Then, the cells were mixed with a mixture of cell cryopreservation medium CP-1, HSA (Albuminar) and RPMI, and cryopreserved at -80°C until use.

### (2) Immunophenotype of transduced T cells

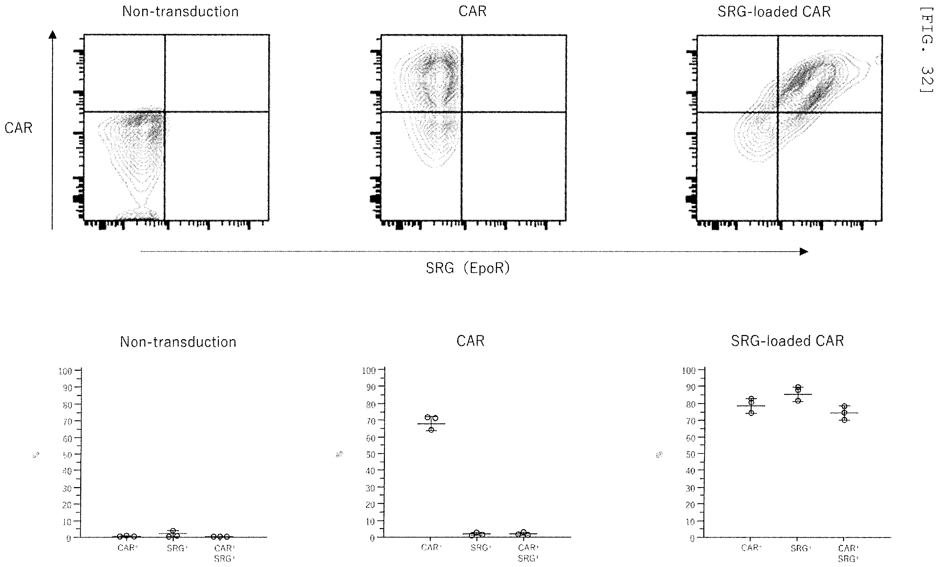

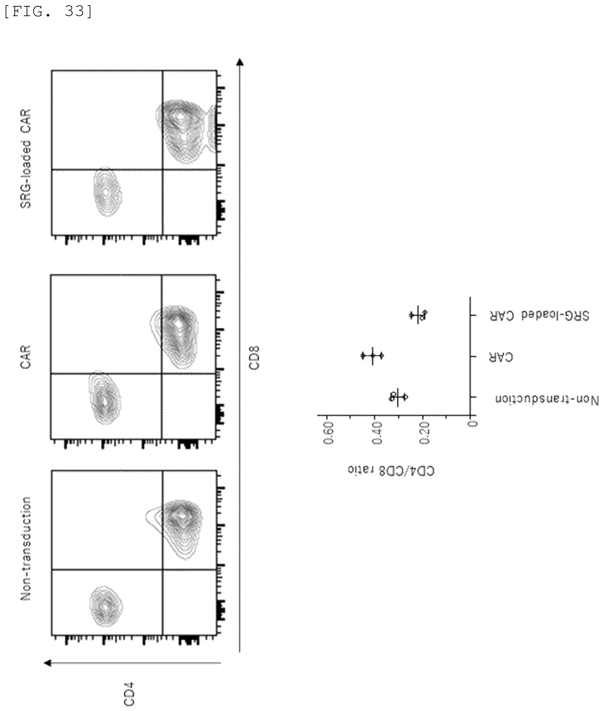

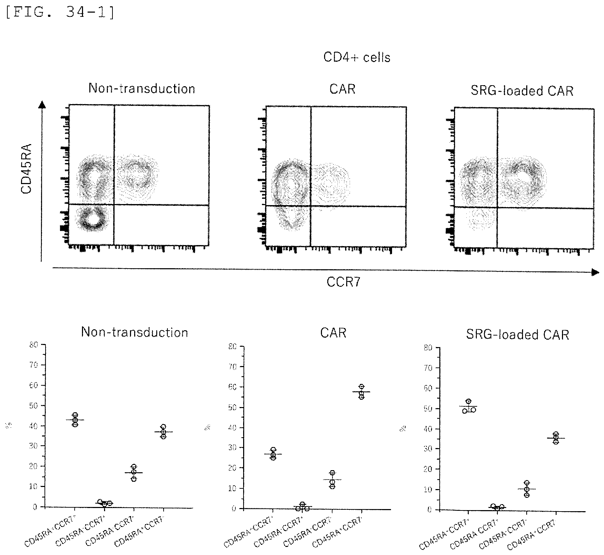

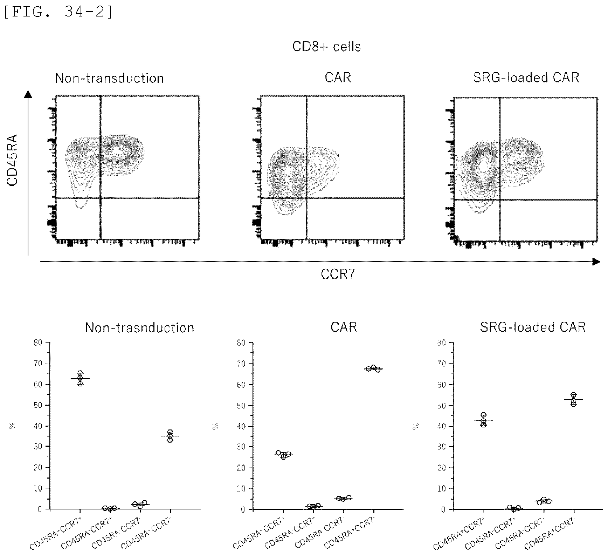

The cryopreserved transduced T cells were thawed, stained with monoclonal antibodies specific for CAR (Cytoart, Inc.) and SRG (EpoR) (R&D Systems, Inc.), and stained with monoclonal antibodies specific for CD45RA and CCR7 on CD4-positive or CD8-positive cells (BioLegend, Inc.), and subjected to flow cytometry analysis. FIG. 32 shows CAR and SRG (EpoR) positivity rates. FIG. 33 shows ratios of CD4 to CD8 (CAR-T cells are CAR-positive cells, and SRG-loaded CAR-T cells are a cell population positive for both CAR and SRG). FIG. 34-1 and FIG. 34-2 show rates of CD4 and CD8 T cell subsets (CAR-T cells are CAR-positive cells, and SRG-loaded CAR-T cells are a cell population positive for both CAR and SRG). SRG-loaded CAR-T cells maintained the CD45RA-positive CCR7-positive T cell subset as compared with CAR-T cells.

### Example 9. In Vitro cytotoxicity of SRG-loaded CAR-T cell (1) Measurement of cytotoxic activity

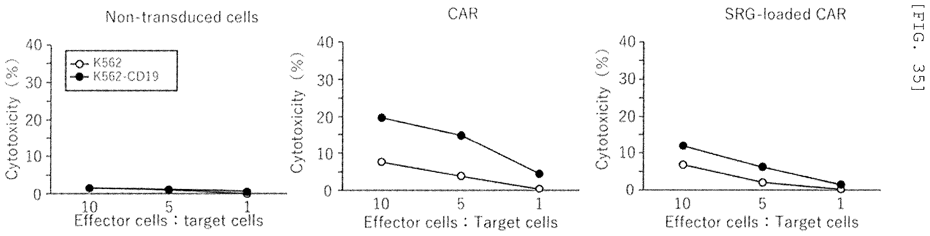

The transduced T cells prepared and cryopreserved in Example 8 were thawed and mixed with K562 or K562-CD19 cells at an effector:target ratio of 10:1, 5:1, or 1:1 in a V-bottom 96-well microplate, and co-cultured in an incubator at 37°C and 5% CO₂ for 18 hours. The microplate was centrifuged at 250 x g at room temperature for 10 minutes. After a supernatant was dispensed into a flat-bottom 96-well microplate at 100 µL/well, the cytotoxicity was measured using LDH Cytotoxicity Detection Kit (TAKARA BIO INC.). As shown in FIG. 35, though the intensity of the cytotoxicity of SRG-loaded CAR-T cells was slightly weaker than that of CAR-T cells, SRG-loaded CAR-T cells exhibited cytotoxicity against CD19-positive cells.

### (2) Production of IL-2 and IFN-γ by CAR-transduced T cells

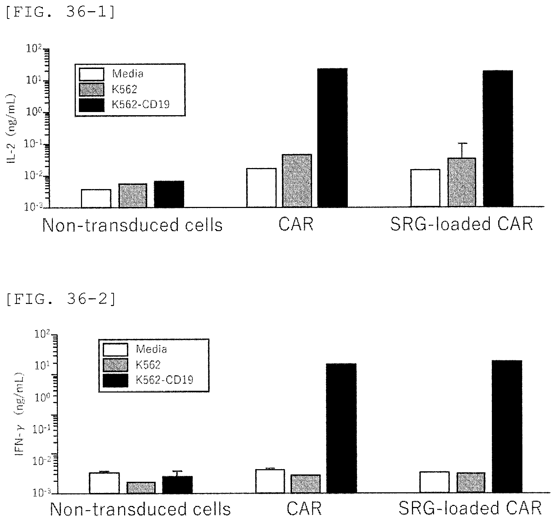

The transduced cells prepared and cryopreserved in Example 8 were thawed, mixed with K562 or K562-CD19 cells at an effector:target ratio of 5:1 in CTL-Test PLUS Medium (Cellular Technology Limited), and transferred to a V-bottom 96-well microplate. The cells were then co-cultured for 18 hours in a 37°C, 5% CO₂ incubator. After the microplate was centrifuged at 250 x g at room temperature for 10 minutes, the supernatant was separated and used to measure the production of IL-2 and IFN-γ using a Human ProQuantum Immunoassay Kit (Invitrogen). As shown in FIG. 36-1 and FIG. 36-2, it was shown that SRG-loaded CAR-T cells were activated in response to CD19-positive cells and produced cytokines to the same extent as CAR-T cells.

### Example 10. In vivo antitumor effect of SRG-loaded CAR-T cell in tumor-bearing mouse

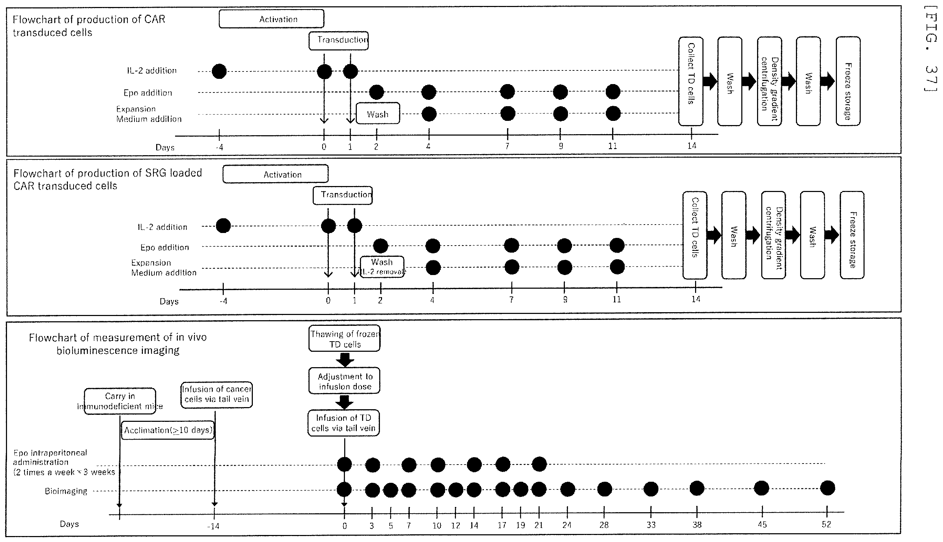

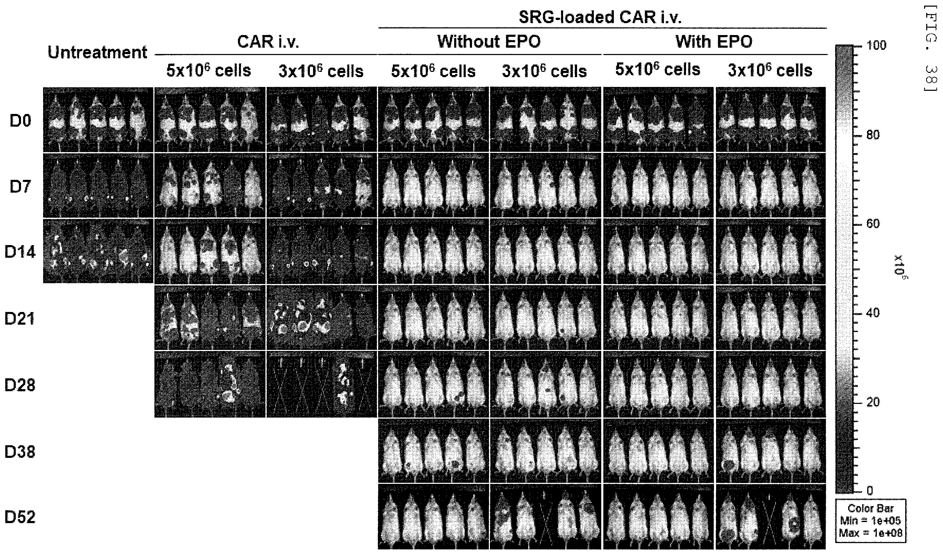

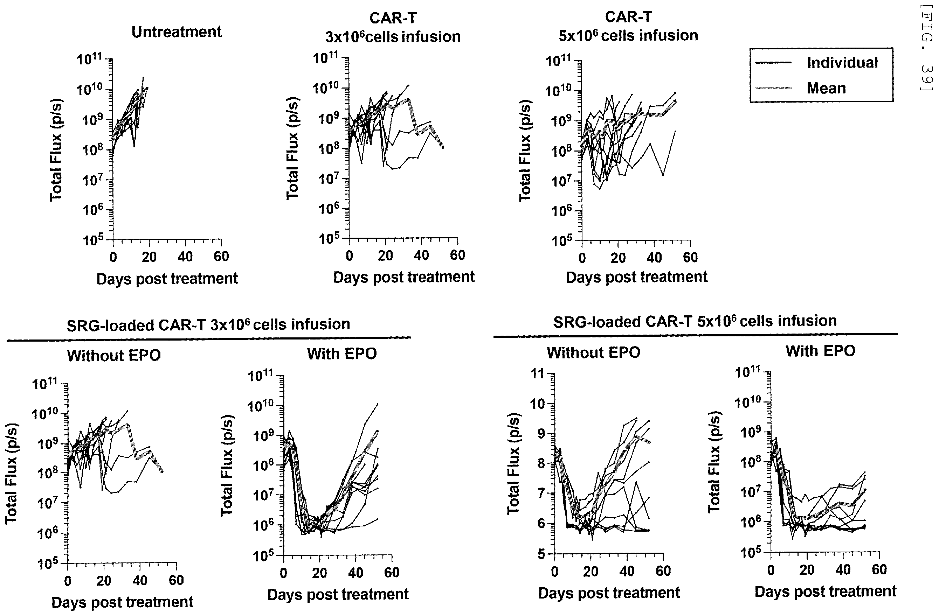

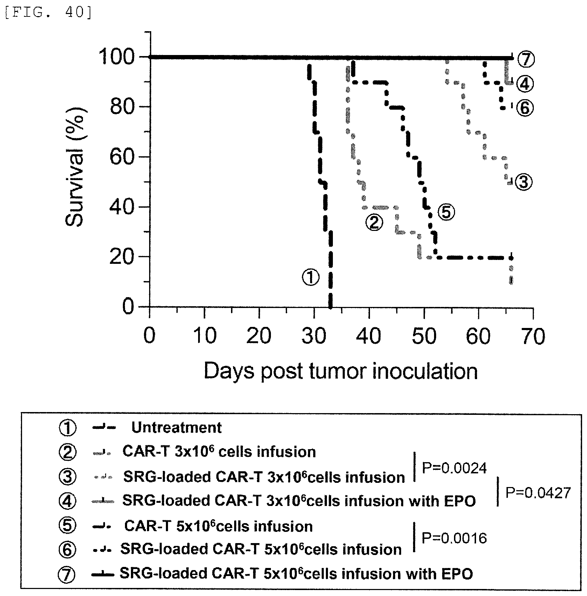

As shown in FIG. 37, immunodeficient NOG mice were inoculated with 1 × 10⁶ cells of human CD19-positive acute lymphoblastic leukemia cell line NALM-6 transduced with the Emerald luciferase gene via the tail vein, and 14 days later, infused with 3 × 10⁶ or 5 × 10⁶ CAR-transduced cells or SRG-loaded CAR-T cells prepared in Example 8 (n=10 for each). To the mice, 90 IU/injection of EPO was administered intraperitoneally (twice a week for 3 weeks), or EPO was not administered. *In vivo* bioluminescence imaging of luciferase activity of tumor cells over time after transfusion of the transduced T cells is shown in FIG. 38, graphs of the luminescence intensity quantified are shown in FIG. 39, and Kaplan-Meier curves are shown in FIG. 40. SRG-loaded CAR-T cells showed higher antitumor activity *in vivo* than CAR-T cells did. Furthermore, the groups receiving SRG-loaded CAR-T cell infusion in combination with EPO showed stronger antitumor activity and better overall survival rates than the other groups.

### Example 11. Preparation of SRG-FFF40 construct

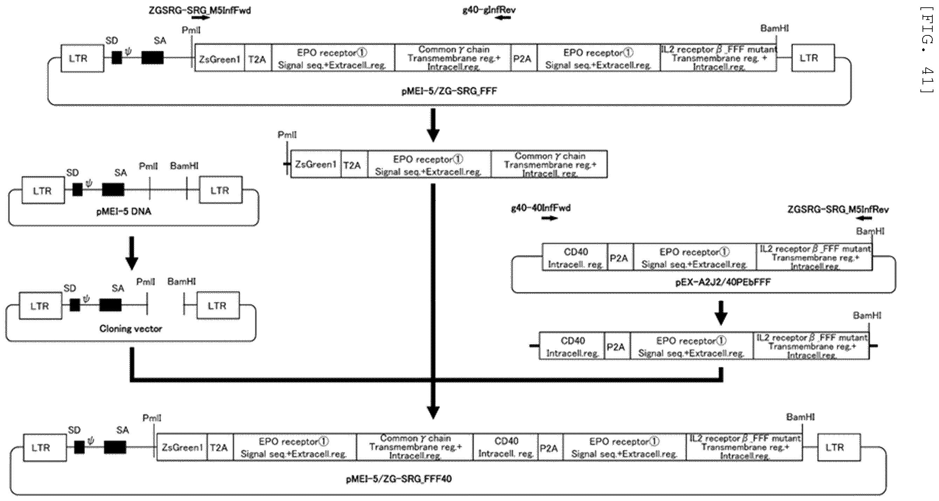

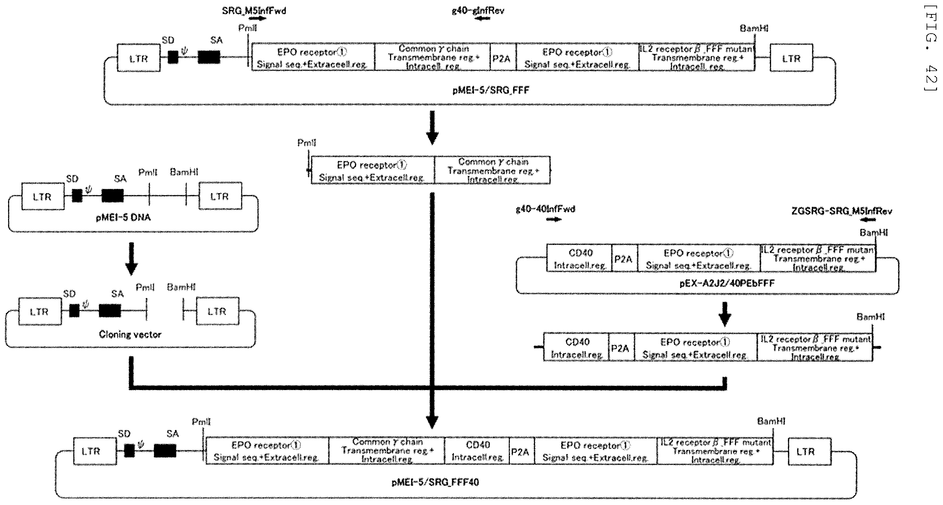

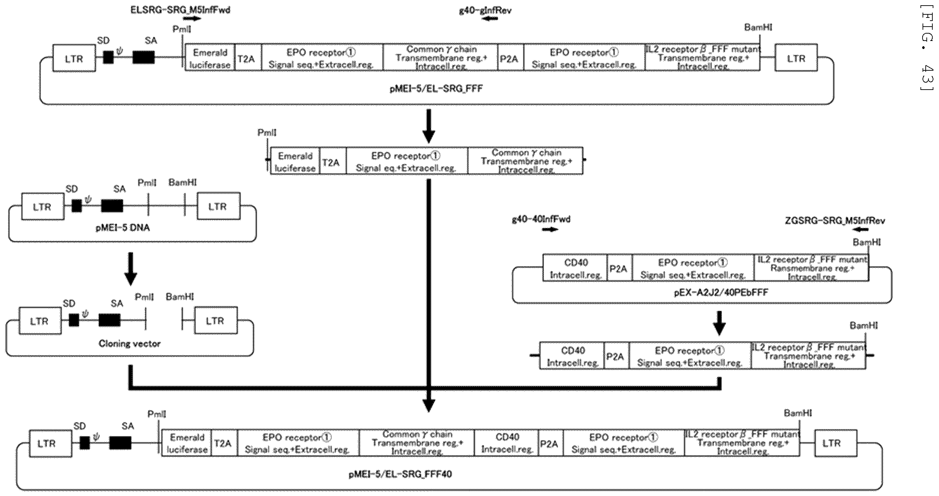

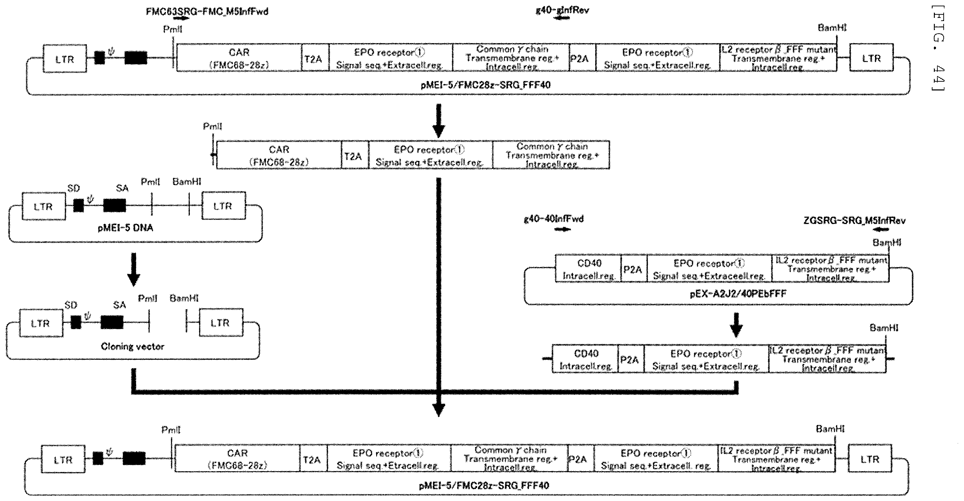

As shown in FIG. 41, retroviral plasmid pMEI-5/ZG-SRG_FFF40 was prepared so that a nucleotide sequence (SEQ ID NO: 72) encoding a polypeptide (SEQ ID NO: 71) from position 216 to position 277 (including an intracellular domain) in the human CD40 amino acid sequence shown by NCBI Reference Sequence: NP_001241.1 was fused downstream of IL-2Rγ. In addition, as shown in FIG. 42, retroviral plasmid pMEI-5/SRG_FFF40, which does not contain ZsGreen, was prepared. Furthermore, as shown in FIG. 43, retroviral plasmid pMEI-5/EL-SRG_FFF40, a version containing Emerald luciferase, was prepared. Furthermore, as shown in FIG. 44, retroviral plasmid pMEI-5/FMC28z-SRG_FFF40, which contains the FMC63-28Z chimeric antigen receptor (SEQ ID NO:57), was prepared.

### Example 12. In vitro cytotoxicity of SRG-FFF40-loaded CAR-T cell

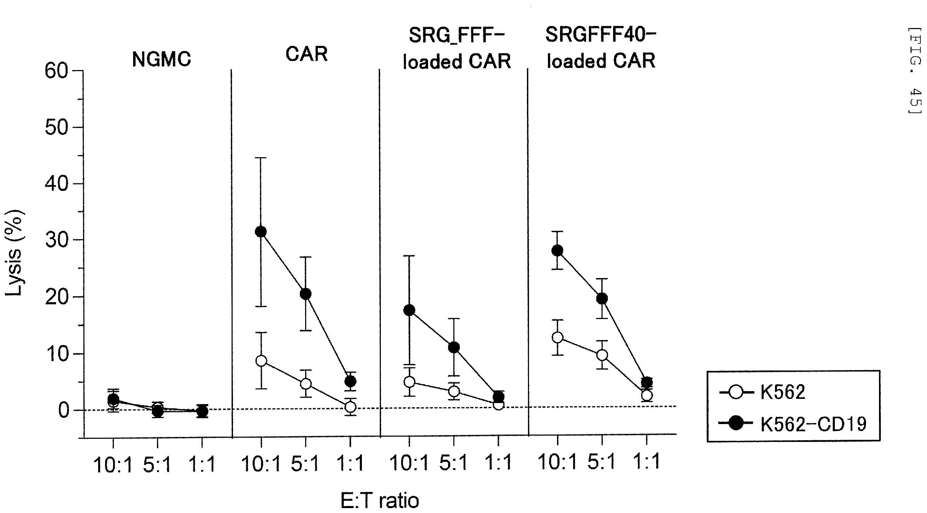

The cytotoxic activity of SRG-FFF40-loaded CAR-T cells was measured using the same procedure as described in Example 9-(1). As shown in FIG. 45, the cytotoxic activity of SRG-FFF40-loaded CAR-T cells was stronger than that of SRG-FFF-loaded CAR-T cells and was equivalent to that of CAR-T cells.

### Example 13. In vivo antitumor effect of SRG-FFF40-loaded CAR-T cell in tumor-bearing mouse

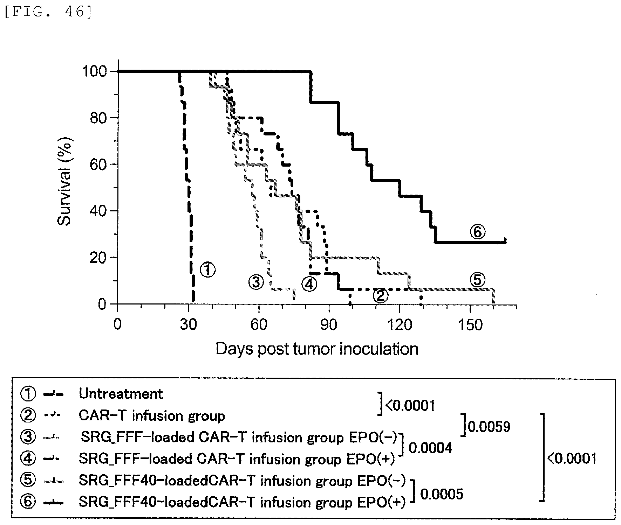

The *in vivo* antitumor effect of SRG-FFF40-loaded CAR-T cells on tumor-bearing mice was measured using the same procedure as in Example 10. The number of infused cells was 5 × 10⁶ cells (n=15 for each). Kaplan-Meier curves are shown in FIG. 46. SRG-FFF40-loaded CAR-T cells showed higher antitumor activity *in vivo* than CAR-T cells did. Furthermore, the group receiving SRG-FFF40-loaded CAR-T cell infusion in combination with EPO administration showed stronger antitumor activity and a better overall survival rate than the other groups.

### INDUSTRIAL APPLICABILITY

The present invention provides a chimeric receptor comprising a first polypeptide and a second polypeptide, the first polypeptide comprising (i) a first extracellular region that binds to a ligand, (ii) a first transmembrane region, and (iii) a first intracellular region derived from an IL-2 receptor β chain, wherein a mutation is introduced at one or more tyrosine residues in the first intracellular region; and the second polypeptide comprising (iv) a second extracellular region that binds to a ligand, (v) a second transmembrane region, and (vi) a second intracellular region derived from an IL-2 receptor γ chain. Also provided are a nucleic acid encoding the chimeric receptor, a vector containing the nucleic acid, a cell expressing the chimeric receptor, a method for producing the cell, a pharmaceutical composition comprising the cell as an active ingredient, and a method for preventing or treating a disease, the method comprising administering the cell or the pharmaceutical composition to a subject. The methods of the present invention are particularly useful for medical applications.

### SEQUENCE LISTING FREE TEXT

SEQ ID NO: 1: TEG amino acid sequence
SEQ ID NO: 2: TEG nucleic acid sequence
SEQ ID NO: 3: PEB amino acid sequence
SEQ ID NO: 4: PEB nucleic acid sequence
SEQ ID NO: 5: TEGPEB amino acid sequence
SEQ ID NO: 6: TEGPEB nucleic acid sequence
SEQ ID NO: 7: pEX-V_InfRev primer
SEQ ID NO: 8: pEX-V_InfFwd primer
SEQ ID NO: 9: TSRG1_pEXInfFwd primer
SEQ ID NO: 10: TSRG1_pEXInfRev primer
SEQ ID NO: 11: PSRG2_pEXInfFwd primer
SEQ ID NO: 12: PSRG2_pEXInfRev primer
SEQ ID NO: 13: ZG-SRG_YYY amino acid sequence
SEQ ID NO: 14: ZG-SRG_YYY nucleic acid sequence
SEQ ID NO: 15: ZGSRG-ZG_M5InfFwd primer
SEQ ID NO: 16: ZGSRG-ZG_M5InfRev primer
SEQ ID NO: 17: ZGSRG-SRG_M5InfFwd primer
SEQ ID NO: 18: ZGSRG-SRG_M5InfRev primer
SEQ ID NO: 19: ZG-SRG_FYY amino acid sequence
SEQ ID NO: 20: ZG-SRG_FYY nucleic acid sequence
SEQ ID NO: 21: FYYmut_Rev primer
SEQ ID NO: 22: FYYmut_Fwd primer
SEQ ID NO: 23: ZG-SRG_YFY amino acid sequence
SEQ ID NO: 24: ZG-SRG_YFY nucleic acid sequence
SEQ ID NO: 25: YFYmut_Rev primer
SEQ ID NO: 26: YFYmut_Fwd primer
SEQ ID NO: 27: ZG-SRG_YYF amino acid sequence
SEQ ID NO: 28: ZG-SRG_YYF nucleic acid sequence
SEQ ID NO: 29: YYFmut_Rev primer
SEQ ID NO: 30: YYFmut_Fwd primer
SEQ ID NO: 31: ZG-SRG_FFY amino acid sequence
SEQ ID NO: 32: ZG-SRG_FFY nucleic acid sequence
SEQ ID NO: 33: FFYmut_Rev primer
SEQ ID NO: 34: FFYmut_Fwd primer
SEQ ID NO: 35: ZG-SRG_YFF amino acid sequence
SEQ ID NO: 36: ZG-SRG_YFF nucleic acid sequence
SEQ ID NO: 37: YFFmut_Rev primer
SEQ ID NO: 38: YFFmut_Fwd primer
SEQ ID NO: 39: ZG-SRG_FYF amino acid sequence
SEQ ID NO: 40: ZG-SRG_FYF nucleic acid sequence
SEQ ID NO: 41: FYFmut_Rev primer
SEQ ID NO: 42: FYFmut_Fwd primer
SEQ ID NO: 43: ZG-SRG_FFF amino acid sequence
SEQ ID NO: 44: ZG-SRG_FFF nucleic acid sequence
SEQ ID NO: 45: FFFmut_Rev primer
SEQ ID NO: 46: FFFmut_Fwd primer
SEQ ID NO: 47: SRG_YYY amino acid sequence
SEQ ID NO: 48: SRG_YYY nucleic acid sequence
SEQ ID NO: 49: SRG_M5InfFwd primer
SEQ ID NO: 50: SRG_FFF amino acid sequence
SEQ ID NO: 51: SRG_FFF nucleic acid sequence
SEQ ID NO: 52: EL-SRG_FFF amino acid sequence
SEQ ID NO: 53: EL-SRG_FFF nucleic acid sequence
SEQ ID NO: 54: ELSRG-EL_M5InfFwd primer
SEQ ID NO: 55: ELSRG-EL_M5InfRev primer
SEQ ID NO: 56: ELSRG-SRG_M5InfFwd primer
SEQ ID NO: 57: FMC28z amino acid sequence
SEQ ID NO: 58: FMC28z nucleic acid sequence
SEQ ID NO: 59: FMC28z-SRG_FFF amino acid sequence
SEQ ID NO: 60: FMC28z-SRG_FFF nucleic acid sequence
SEQ ID NO: 61: FMC63SRG-FMC_M5InfFwd primer
SEQ ID NO: 62: FMC63SRG-FMC_M5InfRev primer
SEQ ID NO: 63: FMC63SRG-SRG_M5InfFwd primer
SEQ ID NO: 64: EpoRm amino acid sequence
SEQ ID NO: 65: EpoRm nucleic acid sequence
SEQ ID NO: 66: ZG-EpoRm amino acid sequence
SEQ ID NO: 67: ZG-EpoRm nucleic acid sequence
SEQ ID NO: 68: ZGEpoRm-ZG_MSInfRev primer
SEQ ID NO: 69: ZGEpoRm-EpoRm_M5InfFwd primer
SEQ ID NO: 70: ZGEpoRm-EpoRm_MSInfRev primer
SEQ ID NO: 71: CD40 partial amino acid sequence
SEQ ID NO: 72: CD40 partial nucleic acid sequence
SEQ ID NO: 73: Wild-type human IL-2 receptor subunit beta precursor amino acid sequence
SEQ ID NO: 74: Wild-type human IL-2 receptor subunit gamma precursor amino acid sequence

## Claims

1. A chimeric receptor comprising a first polypeptide and a second polypeptide, the first polypeptide comprising:
(i) a first extracellular region that binds to a ligand,
(ii) a first transmembrane region, and
(iii) a first intracellular region derived from an IL-2 receptor β chain, wherein a mutation is introduced at one or more tyrosine residues in the first intracellular region; and
the second polypeptide comprising:
(iv) a second extracellular region that binds to a ligand,
(v) a second transmembrane region, and
(vi) a second intracellular region derived from an IL-2 receptor γ chain.

2. The chimeric receptor according to claim 1, wherein the one or more tyrosine residues in the first intracellular region derived from an IL-2 receptor β chain described in (iii) are selected from the group consisting of tyrosine residues corresponding to positions 381, 384 and 387 in the amino acid sequence of an IL-2 receptor β chain shown by SEQ ID NO: 73 (RefSeq NP_000869.1).

3. The chimeric receptor according to claim 2, wherein the one or more tyrosine residues in the first intracellular region derived from an IL-2 receptor β chain described in (iii) are tyrosine residues corresponding to positions 381, 384 and 387 in the amino acid sequence of an IL-2 receptor β chain shown by SEQ ID NO: 73 (RefSeq NP_000869.1).

4. The chimeric receptor according to any one of claims 1 to 3, wherein the mutation is a substitution of the tyrosine residue with a phenylalanine residue.

5. The chimeric receptor according to any one of claims 1 to 4, wherein the first extracellular region that binds to a ligand of (i) and/or the second extracellular region that binds to a ligand of (iv) is an extracellular region derived from an erythropoietin receptor.

6. The chimeric receptor according to any one of claims 1 to 5, wherein the first transmembrane region of (ii) is a transmembrane region derived from an IL-2 receptor β chain, and the second transmembrane region of (v) is a transmembrane region derived from an IL-2 receptor γ chain.

7. The chimeric receptor according to any one of claims 1 to 6, wherein the second polypeptide further comprises an intracellular region derived from CD40.

8. A nucleic acid encoding the chimeric receptor according to any one of claims 1 to 7.

9. A vector comprising the nucleic acid of claim 8.

10. A cell expressing the chimeric receptor according to any one of claims 1 to 7.

11. The cell according to claim 10, which further expresses a chimeric antigen receptor or a foreign T cell receptor that recognizes a target cell.

12. A pharmaceutical composition comprising the cell according to claim 10 or 11 as an active ingredient.

13. A method for preventing or treating a disease, the method comprising administering the pharmaceutical composition according to claim 12 to a subject.
